(19) Europäisches Patentamt
European Patent Office
Office européen des brevets

(11) EP 3 438 146 B1

(12) **EUROPEAN PATENT SPECIFICATION**

(45) Date of publication and mention of the grant of the patent:
**08.04.2026 Bulletin 2026/15**

(21) Application number: **17774912.4**

(22) Date of filing: **27.03.2017**

(51) International Patent Classification (IPC):
***C08G 8/28*** (2006.01) ***C08J 5/24*** (2006.01)
***C09J 11/06*** (2006.01) ***C09J 201/00*** (2006.01)
***H01L 23/29*** (2006.01) ***H01L 23/31*** (2006.01)
***H05K 1/03*** (2006.01) ***C07D 333/16*** (2006.01)

(52) Cooperative Patent Classification (CPC):
**C08J 5/244; C07D 333/16; C08G 8/28; C08J 5/249; C09J 11/06; C09J 201/00; H05K 1/03; H10W 74/10; H10W 74/40**

(86) International application number:
**PCT/JP2017/012347**

(87) International publication number:
**WO 2017/170375 (05.10.2017 Gazette 2017/40)**

# (54) CYANIC ACID ESTER COMPOUND AND METHOD FOR PRODUCING SAME, RESIN COMPOSITION, CURED ARTICLE, PREPREG, SEALING MATERIAL, FIBER-REINFORCED COMPOSITE MATERIAL, ADHESIVE AGENT, METAL FOIL-CLAD LAMINATE PLATE, RESIN SHEET, AND PRINTED WIRING BOARD

CYANSÄUREESTERVERBINDUNG UND VERFAHREN ZUR HERSTELLUNG DAVON, HARZZUSAMMENSETZUNG, GEHÄRTETER ARTIKEL, PREPREG, DICHTUNGSMATERIAL, FASERVERSTÄRKTER VERBUNDSTOFF, HAFTMITTEL, MIT METALLFOLIE BESCHICHTETE LAMINATPLATTE, HARZFOLIE UND LEITERPLATTE

COMPOSÉ D’ESTER D’ACIDE CYANIQUE ET SON PROCÉDÉ DE PRODUCTION, COMPOSITION DE RÉSINE, ARTICLE DURCI, PRÉIMPRÉGNÉ, MATÉRIAU D’ÉTANCHÉITÉ, MATÉRIAU COMPOSITE RENFORCÉ PAR DES FIBRES, AGENT ADHÉSIF, PLAQUE STRATIFIÉE REVÊTUE D’UNE FEUILLE MÉTALLIQUE, FEUILLE DE RÉSINE ET CARTE DE CIRCUIT IMPRIMÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2016 JP 2016070165**
**31.03.2016 JP 2016070166**
**31.03.2016 JP 2016070167**

(43) Date of publication of application:
**06.02.2019 Bulletin 2019/06**

(73) Proprietor: **Mitsubishi Gas Chemical Company, Inc.**
**Tokyo 100-8324 (JP)**

(72) Inventors:
- **KOGA, Shota**
  **Tokyo 125-8601 (JP)**
- **TAKANO, Kentaro**
  **Tokyo 125-8601 (JP)**
- **KATAGIRI, Masayuki**
  **Niigata-shi**
  **Niigata 950-3112 (JP)**
- **YASUDA, Yoshihiro**
  **Niigata-shi**
  **Niigata 950-3112 (JP)**
- **TSUJIMOTO, Tomoo**
  **Tokyo 100-8324 (JP)**

(74) Representative: **Hoffmann Eitle**
**Patent- und Rechtsanwälte PartmbB**
**Arabellastraße 30**
**81925 München (DE)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:

EP-A1- 1 942 129
EP-A1- 2 634 205
EP-A1- 2 671 904
WO-A1-2016/024425
JP-A- 2000 319 345
JP-A- 2001 064 339
JP-A- 2012 131 945
JP-A- 2013 006 955
JP-A- H10 237 060
JP-A- H10 237 060
US-A1- 2014 363 958

Remarks:

The file contains technical information submitted after the application was filed and not included in this specification

## Description

Technical Field

The present invention relates to a cyanic acid ester compound, a method for producing the same, a resin composition, and use thereof.

Background Art

Cyanic acid ester compounds form triazine rings by curing and are widely used as starting materials for various functional polymer materials such as composite materials for structures, adhesives, electrical insulating materials, and electric and electronic components, because of their high heat resistance and excellent electrical properties. In recent years, however, stricter physical properties have been demanded for functional polymer materials with a higher level of required performance in these fields of application. Examples of such physical properties include flame retardance, heat resistance, low rates of thermal expansion, low water absorbability, low permittivity, low dielectric loss tangent, weather resistance, chemical resistance, and high fracture toughness. However, these required physical properties have not always been satisfied so far.

The high integration or miniaturization of semiconductor packages widely used in electronics, communication devices, personal computers, etc. has been increasingly accelerated in recent years. In response to this, stricter properties have been required for laminates for semiconductor packages for use in printed circuit boards. Examples of the required properties include properties such as low water absorbability, moisture-absorbing heat resistance, flame retardance, low permittivity, low dielectric loss tangent, low rates of thermal expansion, heat resistance, chemical resistance, and high plating peel strength. However, these required properties have not always been satisfied so far.

For example, in the field of semiconductor packaging materials, undesired warpage occurs due to the mismatched coefficient of thermal expansion between semiconductor chips and substrate materials with thinning of substrates. As an approach for solving this problem, the functional polymer materials themselves for use in substrate materials are required to have lower thermal expansion and higher heat resistance. Furthermore, use of leadfree solder is promoted for the soldering of printed circuit boards, in consideration of human bodies and environments. In response to this, the functional polymer materials themselves are also required to have lower thermal expansion and higher heat resistance because of being capable of resisting a reflow step at a high temperature.

Conventional functional polymer materials may be allowed to contain a halogen atom or a phosphorus atom from the viewpoint of enhancing the flame retardance of the functional polymer materials. However, the halogen atom has the possibility of generating halogenated gases, which might cause environmental pollution, during combustion. In addition, the halogen atom reduces the insulating properties of final products. Also, the phosphorus atom often reduces the required physical properties (heat resistance, moisture resistance, and low water absorbability, etc.) except for flame retardance. Accordingly, there is also a demand for improving the flame retardance of the functional polymer materials without containing a halogen atom and a phosphorus atom.

In the case of producing a laminate for use in printed circuit boards, etc., first, monomers before curing are dissolved as functional polymer material precursors in a solvent such as methyl ethyl ketone to prepare varnish. Then, glass fiber is impregnated with this varnish and dried to prepare a prepreg. Therefore, there is also a demand for improving the solvent solubility of the monomers.

In the field of semiconductor encapsulation materials, studies have been actively conducted to replace silicon (Si) semiconductor devices with wide-gap semiconductors such as silicon carbide (SiC) semiconductors with the aim of reduction in power loss (energy saving). The SiC semiconductors are more chemically stable than the Si semiconductors and therefore permit operation at a high temperature exceeding 200°C. Thus, it can also be expected that apparatuses are miniaturized. In response to this, compositions comprising functional polymer materials for use in encapsulation materials are required to have heat resistance, low thermal expansion, and heat resistance at high temperatures over a long period (hereinafter, referred to as long-term heat resistance), etc.

Use of a bifunctional cyanatophenyl-based cyanic acid ester compound in which hydrogen of a methylene group that bonds cyanatophenyl groups is replaced with a particular alkyl group (1,1-bis(4-cyanatophenyl)isobutane) has been proposed as an example of obtaining a cured product of a cyanic acid ester compound alone which possesses low thermal expansion and heat resistance (see Patent Literature 1). However, for the bifunctional cyanatophenyl-based cyanic acid ester compound, the flame retardance (persistency at high temperatures) is reduced by replacing hydrogen of the methylene group that bonds cyanatophenyl groups with an alkyl group. Moreover, Patent Literature 1 has no mention about flame retardance and long-term heat resistance.

Use of a cyanic acid ester compound having an aralkyl structure has been proposed as an example of obtaining a cured product of a cyanic acid ester compound alone which possesses low thermal expansion and flame retardance (see Patent Literature 2). However, the cyanic acid ester compound having an aralkyl structure is poorly soluble in a solvent and

is thus difficult to handle.

Use of an isocyanuric acid skeleton-containing cyanic acid ester compound (see Patent Literature 3), a triazine skeleton-containing cyanic acid ester compound (see Patent Literature 4), a bifunctional cyanatophenyl-based cyanic acid ester compound in which hydrogen of a methylene group that bonds cyanatophenyl groups is replaced with a biphenyl group (see Patent Literature 5), and a cyanation product of a phenol-modified xylene formaldehyde resin (see Patent Literature 6), a combination of a trifunctional cyanatophenyl-based (trisphenolalkane-based) cyanic acid ester compound and a bifunctional cyanatophenyl-based cyanic acid ester compound (see Patent Literature 7), and a combination of a bisphenol A-based cyanic acid ester compound and an imide skeleton-containing cyanic acid ester compound (see Patent Literature 8) have been proposed as examples of obtaining a cured product of a cyanic acid ester compound alone which possesses flame retardance and/or heat resistance. However, all of these literatures have no mention about the rate of thermal expansion, long-term heat resistance, and/or solvent solubility.

Composition comprising a novolac-based cyanate, a novolac-based phenol resin, and an inorganic filler has been proposed as a composition containing a cyanic acid ester compound and possessing heat resistance and heat resistance at high temperatures over a long period (Patent Literature 9). However, a test on the long-term heat resistance in Patent Literature 9 was conducted for approximately 50 hours (250°C), which is not sufficient as a test time. Moreover, this literature has no mention about the rate of thermal expansion and solvent solubility.

Cyanic acid ester compounds have heretofore been known as resins for printed circuit boards excellent in heat resistance and electrical properties. Resin compositions containing bisphenol A-based cyanic acid ester compounds, other thermosetting resins, and the like are widely used as printed circuit board materials, etc. The bisphenol A-based cyanic acid ester compounds have excellent properties such as electrical properties, mechanical properties, and chemical resistance, but may be insufficient in terms of low water absorbability, moisture-absorbing heat resistance, flame retardance, or heat resistance. Therefore, various cyanic acid ester compounds differing in structure have been studied for the purpose of further improving properties.

Novolac-based cyanic acid ester compounds are often used as resins structurally different from the bisphenol A-based cyanic acid ester compounds (see Patent Literatures 10 and 11). However, problems of the novolac-based cyanic acid ester compounds are that: the novolac-based cyanic acid ester compounds tend to be insufficiently cured; and the resulting cured products have a large rate of water absorption and low moisture-absorbing heat resistance. The formation of prepolymers of the novolac-based cyanic acid ester compounds and the bisphenol A-based cyanic acid ester compounds has been proposed as a method for solving these problems (see Patent Literatures 12 and 13). The formation of such prepolymers improves curability, but is still insufficient for improvement in properties, i.e., low water absorbability, moisture-absorbing heat resistance, and heat resistance. Therefore, there is a demand for further improvement in heat resistance.

For the miniaturization or high density of multilayer printed circuit boards, multiple buildup layers are used in the multilayer printed circuit boards, and the miniaturization and high density of wiring are demanded. In response to this, the thinning of laminates for use in such buildup layers has been actively studied.

A further problem of the multilayer printed circuit boards is expanded warpage. Therefore, resin compositions serving as materials for insulating layers are required to have properties such as high glass transition temperatures and low thermal expansibility.

EP 2 671 904 A1 relates to a curable resin composition. EP 1 942 129 A1 relates to a cyanate ester polymer obtained by polymerizing a cyanate ester compound.

Citation List

Patent Literature

Patent Literature 1: International Publication No. WO 2012/057144
Patent Literature 2: Japanese Patent No. 4407823
Patent Literature 3: Japanese Patent No. 4654770
Patent Literature 4: Japanese Patent Laid-Open No. 2012-036114
Patent Literature 5: Japanese Patent No. 5104312
Patent Literature 6: International Publication No. WO 2013/21869
Patent Literature 7: Japanese Patent No. 2613056
Patent Literature 8: Japanese Patent Laid-Open No. 2010-180147
Patent Literature 9: Japanese Patent Laid-Open No. 2013-053218
Patent Literature 10: International Publication No. WO 2013/065694
Patent Literature 11: Japanese Patent Laid-Open No. 11-124433

Patent Literature 12: International Publication No. WO 2014/203866
Patent Literature 13: Japanese Patent No. 4407823

## Summary of Invention

### Technical Problem

A practical cured product of a cyanic acid ester compound alone that possesses low thermal expansion, flame retardance, and heat resistance at high levels has not yet been obtained by using a cyanic acid ester compound having solvent solubility.

An object of the present invention is to provide a novel cyanic acid ester compound that has excellent solvent solubility and provides a cured product having a low rate of thermal expansion and having excellent flame retardance and heat resistance, and a resin composition comprising the compound, etc.

For example, Patent Literatures 10 and 11 have proposed a resin composition excellent in properties such as adhesion, low water absorbability, moisture-absorbing heat resistance, and insulation reliability, consisting of a cyanic acid ester compound and an epoxy resin. However, this resin composition is still insufficient in terms of adhesion and heat resistance. Therefore, there is a demand for further improvement in properties. Among others, epoxy resins are generally resins excellent in adhesion, flowability, and workability and have thus been studied with the aim of conferring further properties using various cyanic acid ester compounds differing in structure.

Accordingly, another object of the present invention is to provide a resin composition that can achieve a printed circuit board excellent in peel strength, glass transition temperature, rate of thermal expansion, rate of water absorption, and thermal conductivity, and to provide a prepreg and a monolayer or laminated resin sheet comprising the same, and a metal foil-clad laminate and a printed circuit board comprising the prepreg, etc.

A further alternative object of the present invention is to provide a resin composition that can achieve a printed circuit board not only having a high glass transition temperature and low thermal expansibility but being also excellent in flexural modulus and thermal conductivity, and to provide a prepreg and a monolayer or laminated resin sheet comprising the same, and a metal foil-clad laminate and a printed circuit board comprising the prepreg, etc.

### Solution to Problem

The present inventors have found that a cyanic acid ester compound obtained by cyanation of a thiophene-containing phenol novolac resin has excellent solvent solubility and excellent handleability, and a resin composition containing such a cyanic acid ester compound can achieve a cured product, etc. having a low rate of thermal expansion and having excellent flame retardance and heat resistance, reaching the present invention.

The present inventors have conducted diligent studies on the objects and consequently found that a cured product having excellent peel strength, glass transition temperature, rate of thermal expansion, rate of water absorption, and thermal conductivity is obtained by use of a resin composition containing a thiophene-containing phenol novolac-based cyanate, reaching the present invention.

The present inventors have conducted diligent studies on the objects and consequently found that a cured product having a high glass transition temperature and excellent low thermal expansibility, flexural modulus, and thermal conductivity is obtained by use of a resin composition containing a thiophene-containing phenol novolac-based cyanate and a maleimide compound, reaching the present invention.

Specifically, the present invention is as follows:

[1] A cyanic acid ester compound having a structure represented by the following general formula (1):

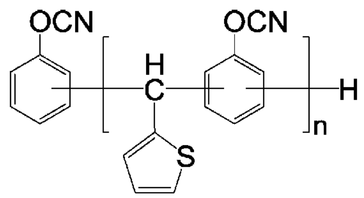 (1)

wherein n represents an integer of 2 to 10.

[2] The cyanic acid ester compound according to [1], wherein the cyanic acid ester compound has a weight-average molecular weight Mw of 100 to 3500.

[3] The cyanic acid ester compound according to the above [1] or [2], wherein the cyanic acid ester compound is a

cyanation product of a thiophene-containing phenol novolac resin represented by the following formula (2):

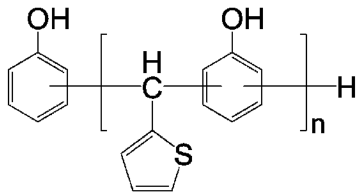

(2)

wherein n represents an integer of 2 to 10.

[4] A method for producing a cyanic acid ester compound, comprising a cyanation step of cyanating a thiophene-containing phenol novolac resin to obtain a cyanic acid ester compound having a structure represented by the following general formula (1):

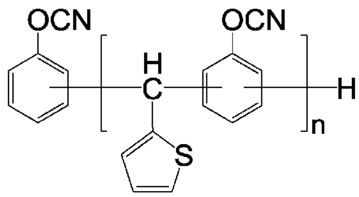

(1)

wherein n represents an integer of 2 to 10.

[5] The method for producing a cyanic acid ester compound according to the above [4], wherein the method further comprises, before the cyanation step, the step of reacting 2-thiophene aldehyde with phenol in the presence of a basic catalyst to obtain the thiophene-containing phenol novolac resin.

[6] A resin composition comprising a cyanic acid ester compound according to any one of the above [1] to [3].

[7] The resin composition according to the above [6], wherein a content of the cyanic acid ester compound is 1 to 90 parts by mass with respect to 100 parts by mass of a resin solid in the resin composition.

[8] The resin composition according to the above [6] or [7], wherein the resin composition further comprises one or more components selected from the group consisting of a cyanic acid ester compound other than the cyanic acid ester compound, a maleimide compound, a phenol resin, an epoxy resin, an oxetane resin, a benzoxazine compound, and a compound having a polymerizable unsaturated group.

[9] The resin composition according to the above [8], wherein the resin composition comprises an epoxy resin.

[10] The resin composition according to the above [8] or [9], wherein the resin composition comprises a maleimide compound.

[11] The resin composition according to any one of the above [6] to [10], wherein the resin composition further comprises a filler.

[12] The resin composition according to the above [11], wherein a content of the filler is 50 to 1600 parts by mass with respect to 100 parts by mass of a resin solid in the resin composition.

[13] A cured product obtained by curing a resin composition according to any one of the above [6] to [12].

[14] A prepreg for a structural materialobtained by impregnating or coating a base material with a resin composition according to any one of the above [6] to [12], followed by drying.

[15] A material for encapsulation comprising a resin composition according to any one of the above [6] to [12].

[16] A fiber-reinforced composite material comprising a resin composition according to any one of the above [6] to [12].

[17] An adhesive comprising a resin composition according to any one of the above [6] to [12].

[18] A prepreg comprising a base material, and a resin composition according to any one of the above [6] to [12] with which the base material is impregnated or coated.

[19] A metal foil-clad laminate comprising at least one or more layers of a prepreg according to the above [18], and metal foil disposed on one side or both sides of the prepreg.

[20] A resin sheet comprising a resin composition according to any one of the above [6] to [12].

A resin sheet comprising a support, and a layer comprising a resin composition according to any one of the above [6] to [12] disposed on a surface of the support.

[21] A printed circuit board comprising an insulating layer, and a conductor layer formed on a surface of the insulating layer, wherein the insulating layer comprises a resin composition according to any one of the above [6] to [12].

Advantageous Effects of Invention

The present invention is highly industrially practical because a prepreg, a resin sheet, a metal foil-clad laminate, etc. excellent in peel strength, glass transition temperature, rate of thermal expansion, rate of water absorption, and thermal conductivity can be obtained, and a high-performance printed circuit board can be achieved by use of this prepreg, resin sheet, or metal foil-clad laminate.

The present invention is highly industrially practical because a prepreg, a resin sheet, a metal foil-clad laminate, etc. having a high glass transition temperature and having excellent thermal expansibility, flexural modulus, and thermal conductivity can be obtained, and a high-performance printed circuit board can thereby be achieved.

Brief Description of Drawings

[Figure 1] Figure 1 shows a GPC chart of a thiophene-containing phenol novolac resin (TiPROH) obtained in Example 1.

[Figure 2] Figure 2 shows a GPC chart of a cyanic acid ester compound (TiPRCN) obtained in Example 1.

[Figure 3] Figure 3 shows FT-IR charts of the thiophene-containing phenol novolac resin (TiPROH) and the cyanic acid ester compound (TiPRCN) obtained in Example 1.

Description of Embodiments

Hereinafter, the mode for carrying out the present invention (hereinafter, referred to as the "present embodiment") will be described in detail. The present embodiment described below is given merely for illustrating the present invention and is not intended to limit the present invention by the contents described below.

In the present specification, the " (meth)acryloyl group" means both of an "acryloyl group" and a "methacryloyl group" corresponding thereto. The "(meth)acrylate" means both of "acrylate" and "methacrylate" corresponding thereto. The "(meth) acrylic acid" means both of "acrylic acid" and "methacrylic acid" corresponding thereto. In the present embodiment, the " resin solid " or the "resin solid in the resin composition" refer to components except for a solvent and an inorganic filler in the resin composition unless otherwise specified. The term "100 parts by mass of a resin solid " refers to 100 parts by mass in total of components except for a solvent and an inorganic filler (G) in the resin composition.

The present embodiment provides a cyanic acid ester compound obtained by cyanation of a thiophene ring-containing phenol novolac resin, and a resin composition comprising the cyanic acid ester compound.

According to another aspect, the present embodiment also provides a cured product obtained by curing the resin composition, and a material for encapsulation, a fiber-reinforced composite material, and an adhesive comprising the resin composition. In the present embodiment, the resin composition is also referred to as a curable resin composition.

[Cyanic acid ester compound]

The cyanic acid ester compound of the present embodiment has a structure represented by the following general formula (1):

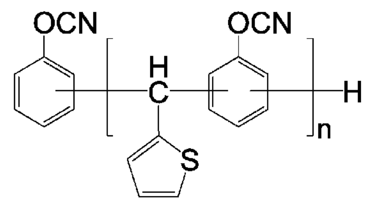 (1)

wherein n represents an integer of 2 to 10. The cyanic acid ester compound may be a mixture of compounds differing in n.

A resin cured product containing the cyanic acid ester compound having such a structure exhibits excellent peel strength, glass transition temperature, rate of thermal expansion, and thermal conductivity.

Also, the resin cured product containing the cyanic acid ester compound having such a structure achieves a high glass transition temperature and excellent low thermal expansibility and flexural modulus and further improves thermal conductivity.

The weight-average molecular weight (Mw) of the cyanic acid ester compound is not particularly limited and is preferably 100 to 3500, more preferably 200 to 3000, further preferably 200 to 2000. When the weight-average molecular weight (Mw) of the cyanic acid ester compound falls within the range described above, the heat resistance tends to be

further improved. The weight-average molecular weight (Mw) can be measured by gel permeation chromatography (GPC).

n is an integer of 2 to 10.

[Method for producing cyanic acid ester compound]

The method for producing the cyanic acid ester compound of the present embodiment may comprise, for example, a cyanation step of cyanating a thiophene-containing phenol novolac resin to obtain the cyanic acid ester compound having a structure represented by the general formula (1) and, if necessary, may further comprise, before the cyanation step, a thiophene-containing phenol novolac resin synthesis step of reacting 2-thiophene aldehyde with phenol in the presence of a basic catalyst to obtain the binaphthol aralkyl resin. Hereinafter, each step will be described in detail.

<Thiophene ring-containing phenol novolac resin synthesis step>

The thiophene-containing phenol novolac resin synthesis step is the step of reacting 2-thiophene aldehyde with phenol in the presence of a basic catalyst to obtain the thiophene-containing phenol novolac resin. This reaction can be performed by a method described in, for example, Japanese Patent Laid-Open No. 10-237060, Japanese Patent No. 4082481, or Japanese Patent No. 4111410.

Examples of the thiophene-containing phenol novolac resin include, but are not particularly limited to, a compound represented by the general formula (2) given below. The obtained thiophene-containing phenol novolac resin may be a mixture of compounds differing in n.

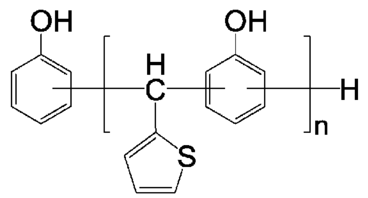 (2)

wherein n represents an integer of 2 to 10. The thiophene-containing phenol novolac resin may be a mixture of compounds differing in n. The range of n is the same as that of n in the formula (1).

Examples of the basic catalyst for use in the production of the thiophene-containing phenol novolac resin include: alkali metal hydroxides such as lithium hydroxide, sodium hydroxide, and potassium hydroxide; alkaline earth metal hydroxides such as magnesium hydroxide and calcium hydroxide; alkali metal alkoxides such as sodium methoxide, sodium ethoxide, potassium methoxide, potassium ethoxide, and potassium-tert-butoxide; and alkaline earth metal alkoxides such as magnesium methoxide and magnesium ethoxide. One type of basic catalyst or two or more types of basic catalysts in combination can be used.

The amount of the basic catalyst used is usually 0.005 to 2.0 mol, preferably 0.01 to 1.1 mol, with respect to 1 mol of the phenol. When the amount of the basic catalyst is 0.005 mol or larger, the progression of the reaction is accelerated so that the reaction tends to be able to proceed at a lower temperature. When the amount of the acidic catalyst is 2.0 mol or smaller, treatment cost for subsequent aftertreatment such as neutralization or purification tends to be able to be further reduced.

If necessary, an inert solvent can also be used in the reaction. Examples of the solvent include organic solvents including: alcohol solvents such as methanol, ethanol, propanol, and isopropanol; and aromatic hydrocarbon solvents such as toluene and xylene. One type of solvent or two or more types of solvents in combination can be used. In the case of using the solvent, the amount of the solvent used is usually 5 to 500 parts by mass, preferably 10 to 300 parts by mass, with respect to 100 parts by mass of the phenol.

The reaction temperature in the reaction is usually 0 to 200°C, preferably 50 to 150°C. The reaction time in the reaction is 1 to 200 hours, preferably 5 to 100 hours. Under such reaction conditions, the progression of the reaction tends to be further accelerated.

In the reaction, the amount of the phenol used is 1.5 to 20 mol, preferably 1.8 to 10 mol, with respect to 1 mol of the 2-thiophene aldehyde.

The reaction is performed by adding a basic catalyst into a mixture of 2-thiophene aldehyde and phenol (and a solvent, if necessary), followed by heating. Alternatively, 2-thiophene aldehyde may be gradually added to a mixture of phenol and a catalyst (and a solvent, if necessary) under heating. The reaction may be performed with stirring, may be performed in air or an inert atmosphere (nitrogen, helium, argon, etc.), and may be performed under normal pressure or increased pressure. The progression of the reaction can be confirmed (or monitored) by high-performance liquid chromatography (HPLC), thing-layer chromatography (TLC), or the like.

After the completion of the reaction, the reaction mixture contains unreacted 2-thiophene aldehyde, unreacted phenol, the basic catalyst, reaction by-products, etc. Therefore, the binaphthol aralkyl resin may be separated and purified using a method routinely used, for example, a separation approach such as neutralization, washing with water, filtration, concentration, extraction, crystallization, recrystallization, or column chromatography, or a combination of these separation approaches.

<Cyanation step>

The cyanation step is the step of cyanating a thiophene-containing phenol novolac resin to obtain the cyanic acid ester compound having a structure represented by the general formula (1). Specifically, this step involves cyanating the hydroxy groups of a thiophene-containing phenol novolac resin represented by the general formula (2) given below to obtain the cyanic acid ester compound having a structure represented by the general formula (1). The thiophene-containing phenol novolac resin for use in the cyanation step may be a mixture of compounds differing in n.

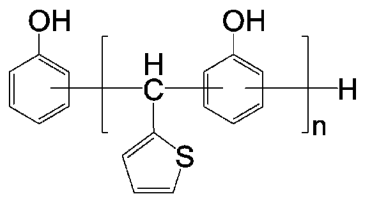 (2)

wherein n represents an integer of 2 to 10. The range of n is the same as that of n in the formula (1).
The cyanation method is not particularly limited, and a method known in the art can be applied thereto.
Examples of such a process include a method which involves obtaining or synthesizing a hydroxy group-containing compound having the desired skeleton, and modifying the hydroxy group by an approach known in the art for cyanation. Examples of the approach for cyanating the hydroxy group include an approach described in Ian Hamerton, "Chemistry and Technology of Cyanate Ester Resins", Blackie Academic & Professional.
Specifically, the cyanic acid ester compound of the present embodiment can be obtained by, for example: a method which involves reacting the thiophene-containing phenol novolac resin with a cyanogen halide in the presence of a basic compound in a solvent; a method which involves reacting the thiophene-containing phenol novolac resin with a cyanogen halide in the presence of a base in a solvent such that the cyanogen halide is always present in excess of the base (U.S. Patent No. 3553244); a method which involves using a tertiary amine as a base, and using this tertiary amine in excess of a cyanogen halide while adding the tertiary amine to the thiophene-containing phenol novolac resin in the presence of a solvent and then adding the cyanogen halide dropwise, or adding dropwise the cyanogen halide and the tertiary amine in combination (Japanese Patent No. 3319061); a method which involves reacting the thiophene-containing phenol novolac resin, a trialkylamine, and a cyanogen halide in a continuous plug flow manner (Japanese Patent No. 3905559); a method which involves reacting the thiophene-containing phenol novolac resin with a cyanogen halide in the presence of a tert-amine in a nonaqueous solution and treating the resulting by-product tertammonium halide with cation and anion exchange pairs (Japanese Patent No. 4055210); a method which involves simultaneously adding a tertiary amine and a cyanogen halide to the thiophene-containing phenol novolac resin in the presence of a solvent separable from water, reacting the mixture, then washing the reaction mixture with water, separating the reaction mixture into aqueous and organic layers, and purifying the reaction product from the obtained solution by precipitation using a secondary or tertiary alcohol or a hydrocarbon as a poor solvent (Japanese Patent No. 2991054); or a method which involves reacting the thiophene-containing phenol novolac resin, a cyanogen halide, and a tertiary amine under acidic conditions in a two-phase solvent of water and an organic solvent (Japanese Patent No. 5026727).
The compound represented by the formula (2) may be produced by a method known in the art or may be obtained as a commercially available product. For example, a thiophene-containing phenol novolac resin manufactured by Nippon Kayaku Co., Ltd. can be suitably used as the commercially available product.
Hereinafter, the method which involves reacting the thiophene-containing phenol novolac resin with a cyanogen halide in the presence of a basic compound in a solvent will be described as an example. In this case, the thiophene-containing phenol novolac resin serving as a reaction substrate is dissolved in advance in either of a cyanogen halide solution or a basic compound solution, and the cyanogen halide solution and the basic compound solution are then contacted with each other.
Examples of the method for contacting the cyanogen halide solution and the basic compound solution with each other (contact method) include: a method (A) of pouring the basic compound solution to the cyanogen halide solution mixed by stirring, a method (B) of pouring the cyanogen halide solution to the basic compound solution mixed by stirring, and a method (C) of supplying the cyanogen halide solution and the basic compound solution either alternately in a continuous manner or at the same time.

Among the methods (A), (B), and (C), the method (A) is preferred because of suppressing side reaction and being able to obtain a more highly pure cyanic acid ester compound at a high yield.
The contact method of the cyanogen halide solution and the basic compound solution may be performed in a semibatch format or a continuous circulation format.
Particularly, in the case of using the method (A), it is preferred to pour the basic compound in divided portions, because the reaction can be completed without allowing the hydroxy groups of the thiophene-containing phenol novolac resin to remain and a more highly pure cyanic acid ester compound can be obtained at a high yield. The number of divisions is not particularly limited and is preferably 1 to 5. The type of the basic compound may be the same or different for each division.
Examples of the cyanogen halide used in the present embodiment include cyanogen chloride and cyanogen bromide. The cyanogen halide used may be a cyanogen halide obtained by a production method known in the art such as a method of reacting hydrogen cyanide or metal cyanide with halogen, or may be a commercially available product. Alternatively, a reaction solution containing the cyanogen halide obtained by reacting hydrogen cyanide or metal cyanide with halogen may be used directly.
In the cyanation step of the present embodiment, the amount of the cyanogen halide used for the thiophene-containing phenol novolac resin is 0.5 to 5 mol, preferably 1.0 to 3.5 mol, with respect to 1 mol of the hydroxy groups of the thiophene-containing phenol novolac resin.
This is because an unreacted thiophene-containing phenol novolac resin is not allowed to remain so that the yield of the cyanic acid ester compound is enhanced.
Examples of the solvent for use in the cyanogen halide solution include: ketone solvents such as acetone, methyl ethyl ketone, methyl isobutyl ketone, cyclohexanone, and cyclopentanone, aliphatic hydrocarbon solvents such as n-hexane, cyclohexane, and isooctane; aromatic hydrocarbon solvents such as benzene, toluene, and xylene, ether solvents such as diethyl ether, dimethyl cellosolve, diglyme, tetrahydrofuran, methyltetrahydrofuran, dioxane, and tetraethylene glycol dimethyl ether, halogenated hydrocarbon solvents such as dichloromethane, chloroform, carbon tetrachloride, dichloroethane, trichloroethane, chlorobenzene, and bromobenzene, alcohol solvents such as methanol, ethanol, isopropanol, methyl cellosolve, and propylene glycol monomethyl ether, aprotic polar solvents such as N,N-dimethylformamide, N-methylpyrrolidone, 1,3-dimethyl-2-imidazolidone, and dimethyl sulfoxide, nitrile solvents such as acetonitrile and benzonitrile, nitro solvents such as nitromethane and nitrobenzene, ester solvents such as ethyl acetate and ethyl benzoate, and a water solvent. According to the reaction substrate, one type of solvent can be used alone, or two or more types of solvents can be used in combination.
Any of organic and inorganic bases can be used as the basic compound for use in the cyanation step of the present embodiment. One of these basic compounds is used alone, or two or more thereof are used in combination.
Examples of the organic base preferably include, particularly, tertiary amines such as trimethylamine, triethylamine, tri-n-butylamine, triamylamine, diisopropylethylamine, diethyl-n-butylamine, methyl-di-n-butylamine, methylethyl-n-butylamine, dodecyldimethylamine, tribenzylamine, triethanolamine, N,N-dimethylaniline, N,N-diethylaniline, diphenylmethylamine, pyridine, diethylcyclohexylamine, tricyclohexylamine, 1,4-diazabicyclo[2.2.2]octane, 1,8-diazabicyclo[5.4.0]-7-undecene, and 1,5-diazabicyclo[4.3.0]-5-nonene. Among these organic bases, trimethylamine, triethylamine, tri-n-butylamine, and diisopropylethylamine are more preferred, and triethylamine is particularly preferred, because of, for example, obtaining the compound of interest at a good yield.
The amount of the organic base used is preferably 0.1 to 8 mol, more preferably 1.0 to 3.5 mol, with respect to 1 mol of the hydroxy groups of the thiophene-containing phenol novolac resin.
This is because an unreacted thiophene-containing phenol novolac resin is not allowed to remain so that the yield of the cyanic acid ester compound is enhanced.
The inorganic base is preferably a hydroxide of an alkali metal. Examples of the hydroxide of an alkali metal include, but are not particularly limited to, sodium hydroxide, potassium hydroxide, and lithium hydroxide generally used industrially. Sodium hydroxide is particularly preferred because of being inexpensively available.
The amount of the inorganic base used is preferably 1.0 to 5.0 mol, more preferably 1.0 to 3.5 mol, with respect to 1 mol of the hydroxy groups of the thiophene-containing phenol novolac resin.
This is because an unreacted thiophene-containing phenol novolac resin is not allowed to remain so that the yield of the cyanic acid ester compound is enhanced.
In the reaction of the present embodiment, the basic compound can be dissolved in a solvent and used as a solution, as mentioned above. An organic solvent or water can be used as the solvent.
In the case of dissolving the thiophene-containing phenol novolac resin in the basic compound solution, the amount of the solvent used in the basic compound solution is preferably 0.1 to 100 parts by mass, more preferably 0.5 to 50 parts by mass, with respect to 1 part by mass of the thiophene-containing phenol novolac resin.
In the case of not dissolving the thiophene-containing phenol novolac resin in the basic compound solution, the amount of the solvent used is preferably 0.1 to 100 parts by mass, more preferably 0.25 to 50 parts by mass, with respect to 1 part by mass of the basic compound.

The organic solvent for dissolving the basic compound is preferably used when the basic compound is an organic base. Examples thereof include: ketone solvents such as acetone, methyl ethyl ketone, and methyl isobutyl ketone; aromatic hydrocarbon solvents such as benzene, toluene, and xylene; ether solvents such as diethyl ether, dimethyl cellosolve, diglyme, tetrahydrofuran, methyltetrahydrofuran, dioxane, and tetraethylene glycol dimethyl ether, halogenated hydrocarbon solvents such as dichloromethane, chloroform, carbon tetrachloride, dichloroethane, trichloroethane, chlorobenzene, and bromobenzene, alcohol solvents such as methanol, ethanol, isopropanol, methyl cellosolve, and propylene glycol monomethyl ether, aprotic polar solvents such as N,N-dimethylformamide, N-methylpyrrolidone, 1,3-dimethyl-2-imidazolidone, and dimethyl sulfoxide, nitrile solvents such as acetonitrile and benzonitrile, nitro solvents such as nitromethane and nitrobenzene, ester solvents such as ethyl acetate and ethyl benzoate, and aliphatic hydrocarbon solvents such as cyclohexane. The organic solvent can be appropriately selected according to the basic compound, the reaction substrate, and the solvent for use in the reaction. One type of organic solvent can be used alone, or two or more types of organic solvents can be used in combination.

The water for dissolving the basic compound is preferably used when the basic compound is an inorganic base. The water is not particularly limited and may be tap water, may be distilled water, or may be deionized water. Distilled water and deionized water containing few impurities are preferred from the viewpoint of efficiently obtaining the cyanic acid ester compound of interest.

When the solvent for use in the basic compound solution is water, it is preferred to use a catalytic amount of an organic base as a surfactant, from the viewpoint of securing a more sufficient reaction rate. Among others, a tertiary amine less likely to cause side reaction is preferred. The tertiary amine may be any alkylamine, arylamine, or cycloalkylamine. Specific examples thereof include trimethylamine, triethylamine, tri-n-butylamine, triamylamine, diisopropylethylamine, diethyl-n-butylamine, methyl-di-n-butylamine, methylethyl-n-butylamine, dodecyldimethylamine, tribenzylamine, triethanolamine, N,N-dimethylaniline, N,N-diethylaniline, diphenylmethylamine, pyridine, diethylcyclohexylamine, tricyclohexylamine, 1,4-diazabicyclo[2.2.2]octane, 1,8-diazabicyclo[5.4.0]-7-undecene, and 1,5-diazabicyclo[4.3.0]-5-nonene. Among these tertiary amines, trimethylamine, triethylamine, tri-n-butylamine, and diisopropylethylamine are more preferred, and triethylamine is particularly preferred, from the viewpoint of solubility in water and from the viewpoint of obtaining the compound of interest at a better yield. One of these tertiary amines is used alone, or two or more thereof are used in combination.

The total amount of the solvents for use in the cyanation step of the present embodiment is preferably **2.5** to 100 parts by mass with respect to 1 part by mass of the thiophene-containing phenol novolac resin from the viewpoint of more uniformly dissolving the thiophene-containing phenol novolac resin and more efficiently producing the cyanic acid ester compound.

In the cyanation step of the present embodiment, the pH of the reaction solution is not particularly limited, and the reaction is preferably performed with the pH kept at lower than 7. When the pH is kept at lower than 7, the formation of by-products such as imidocarbonate and polymerization products of the cyanic acid ester compound is suppressed so that the cyanic acid ester compound can be efficiently produced. A method of adding an acid to the reaction solution is preferred for keeping the pH of the reaction solution at lower than 7. It is more preferred to keep the pH at lower than 7 by adding an acid to the cyanogen halide solution immediately before the cyanation step and by adding an acid to the reaction system while appropriately measuring the pH of the reaction solution using a pH meter during the reaction.

Examples of the acid for use in this operation include: inorganic acids such as hydrochloric acid, nitric acid, sulfuric acid, and phosphoric acid, and organic acids such as acetic acid, lactic acid, and propionic acid.

The reaction temperature in the cyanation step of the present embodiment is preferably -20 to +50°C, more preferably -15 to 15°C, further more preferably -10 to 10°C, from the viewpoint of suppressing the formation of by-products such as imidocarbonate, polymerization products of the cyanic acid ester compound, and dialkyl cyanamide, the condensation of the reaction solution, and, in the case of using cyanogen chloride as the cyanogen halide, the volatilization of the cyanogen chloride.

The reaction pressure in the cyanation step of the present embodiment may be normal pressure or increased pressure. If necessary, an inert gas such as nitrogen, helium, or argon may be blown into the reaction system.

The reaction time is not particularly limited, and the pouring time for the contact methods (A) and (B) and the contact time for the method (C) is preferably 1 minute to 20 hours, more preferably 3 minutes to 10 hours. It is further preferred to then stir the reaction solution for 10 minutes to 10 hours with the reaction temperature maintained.

When the reaction conditions fall within the range as described above, the cyanic acid ester compound of interest is obtained more economically and more industrially.

In the cyanation step, the degree of progression of the reaction can be analyzed by liquid chromatography or IR spectroscopy, etc. Volatile components such as by-products dicyanogen and dialkyl cyanamide can be analyzed by gas chromatography.

After the completion of the reaction, the cyanic acid ester compound of interest can be isolated by usual aftertreatment operation and, if desired, separation and purification operation. Specifically, an organic solvent phase containing the cyanic acid ester compound can be separated from the reaction solution and washed with water, followed by

concentration, precipitation, or crystallization or washed with water, followed by solvent substitution. For the washing, a method using an acidic aqueous solution such as dilute hydrochloric acid can also be adopted in order to remove an excess of amines. The thoroughly washed reaction solution can be dried by a general method using sodium sulfate, magnesium sulfate, or the like in order to remove water therefrom. For the concentration and the solvent substitution, the organic solvent is distilled off by heating to a temperature of 90°C or lower under reduced pressure in order to suppress the polymerization of the cyanic acid ester compound. For the precipitation or the crystallization, a solvent having a low degree of dissolution can be used. For example, a method of adding dropwise an ether solvent, a hydrocarbon solvent (e.g., hexane), or an alcohol solvent to the reaction solution or pouring the reaction solution to the solvent can be adopted. A method of washing the concentrate of the reaction solution or precipitated crystals with an ether solvent, a hydrocarbon solvent (e.g., hexane), or an alcohol solvent can be adopted in order to wash the obtained crude product. Crystals obtained by the concentration of the reaction solution may be redissolved and then recrystallized. The crystallization may be performed by the simple concentration or cooling of the reaction solution.

The obtained cyanic acid ester compound can be identified by a method known in the art such as NMR. The purity of the cyanic acid ester compound can be analyzed by liquid chromatography or IR spectroscopy, etc. Volatile components such as by-products (e.g., dialkyl cyanamide) or residual solvents in the cyanic acid ester compound can be quantitatively analyzed by gas chromatography. Halogen compounds remaining in the cyanic acid ester compound can be identified using a liquid chromatography-mass spectrometer and can also be quantitatively analyzed by potentiometric titration using a silver nitrate solution or by ion chromatography after decomposition by a combustion method. The polymerization reactivity of the cyanic acid ester compound can be evaluated on the basis of the time to gel by a hot plate method or a torque measurement method.

[Resin composition]

The resin composition of the present embodiment comprises the cyanic acid ester compound mentioned above.

In the resin composition of the present embodiment, a content of the cyanic acid ester compound can be appropriately set according to the desired properties, and is not particularly limited. The content is preferably 1 part by mass to 99 parts by mass, more preferably 10 parts by mass to 90 parts by mass, further preferably 20 to 80 parts by mass, still further preferably 30 to 70 parts by mass, even further preferably 40 to 60 parts by mass, with respect to 100 parts by mass of a resin solid in the resin composition. When the content falls within the range of 1 to 90 parts by mass, the resulting resin composition is excellent in peel strength, glass transition temperature, rate of thermal expansion, rate of water absorption, and thermal conductivity.

This resin composition may contain, for example, a cyanic acid ester compound other than the cyanic acid ester compound mentioned above (hereinafter, referred to as an "additional cyanic acid ester compound"), a maleimide compound, a phenol resin, an epoxy resin, an oxetane resin, a benzoxazine compound, and/or a compound having a polymerizable unsaturated group, without impairing the intended properties. Also, this resin composition may optionally further comprise a polymerization catalyst, a filler, a solvent, and other components.

The resin composition of the present embodiment can be suitably used for a cured product, varnish for resin sheet preparation, a prepreg for a structural material, a material for encapsulation, a fiber-reinforced composite material, an adhesive, etc.

Hereinafter, each component will be described.

<Additional cyanic acid ester compound>

The additional cyanic acid ester compound is not particularly limited as long as the compound intramolecularly has an aromatic moiety in which at least one hydrogen atom is replaced with a cyanato group, and is other than the cyanic acid ester compound represented by the general formula (1). Examples thereof include a compound represented by the following general formula (3):

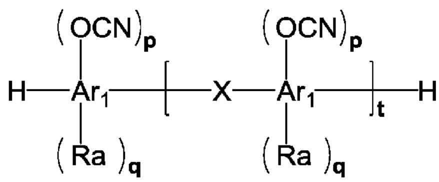 (3)

wherein each $Ar_1$ independently represents a phenylene group, a naphthylene group, or a biphenylene group; a plurality of $Ar_1$ groups may be the same as or different from each other; each Ra independently represents a hydrogen atom, an alkyl group having 1 to 6 carbon atoms, an aryl group having 6 to 12 carbon atoms, an alkoxy group having 1 to 4 carbon atoms,

or a group in which an alkyl group having 1 to 6 carbon atoms is bonded to an aryl group having 6 to 12 carbon atoms; the aromatic ring in Ra optionally has a substituent; the positions of the substituents for $Ar_1$ and Ra can be arbitrarily selected; p represents the number of a cyanato group bonded to $Ar_1$ and each p is independently an integer of 1 to 3; q represents the number of Ra bonded to $Ar_1$ and is 4 - p when $Ar_1$ is a phenylene group, 6 - p when $Ar_1$ is a naphthylene group, and 8 - p when $Ar_1$ is a biphenylene group; t represents the average number of a repeat and is an integer of 0 to 50 (the compound may be a mixture of compounds differing in t); each X independently represents a single bond, a divalent organic group having 1 to 50 carbon atoms (a hydrogen atom may be replaced with a heteroatom), a divalent organic group having 1 to 10 nitrogen atoms (e.g., -N-R-N- (wherein R represents an organic group)), a carbonyl group (-CO-), a carboxy group (-C(=O)O-), a carbonyl dioxide group (-OC(=O)O-), a sulfonyl group ($-SO_2-$), a divalent sulfur atom, or a divalent oxygen atom.

The alkyl group represented by Ra in the general formula (3) may have any of linear, branched, and cyclic structures (e.g., a cycloalkyl group).

A hydrogen atom in the alkyl group or the aryl group represented by Ra in the general formula (3) may be replaced with a halogen atom such as a fluorine atom or a chlorine atom, an alkoxy group such as a methoxy group or a phenoxy group, a cyano group, or the like.

Specific examples of the alkyl group include a methyl group, an ethyl group, a propyl group, an isopropyl group, a n-butyl group, an isobutyl group, a tert-butyl group, a n-pentyl group, a 1-ethylpropyl group, a 2,2-dimethylpropyl group, a cyclopentyl group, a hexyl group, a cyclohexyl group, and a trifluoromethyl group.

Specific examples of the aryl group include a phenyl group, a xylyl group, a mesityl group, a naphthyl group, a phenoxyphenyl group, an ethylphenyl group, an o-, m-, or p-fluorophenyl group, a dichlorophenyl group, a dicyanophenyl group, a trifluorophenyl group, a methoxyphenyl group, and an o-, m-, or p-tolyl group. Examples of the alkoxy group include a methoxy group, an ethoxy group, a propoxy group, an isopropoxy group, a n-butoxy group, an isobutoxy group, and a tert-butoxy group.

Specific examples of the divalent organic group represented by X in the general formula (3) include: alkylene groups such as a methylene group, an ethylene group, a trimethylene group, and a propylene group; cycloalkylene groups such as a cyclopentylene group, a cyclohexylene group, and a trimethylcyclohexylene group; and divalent organic groups having an aromatic ring, such as a biphenylylmethylene group, a dimethylmethylenephenylene-dimethylmethylene group, a fluorenediyl group, and a phthalidodiyl group. A hydrogen atom in the divalent organic group may be replaced with a halogen atom such as a fluorine atom or a chlorine atom, an alkoxy group such as a methoxy group or a phenoxy group, a cyano group, or the like.

Examples of the divalent organic group having 1 to 10 nitrogen atoms represented by X in the general formula (3) include a group represented by -N-R-N-, an imino group, and a polyimide group.

Specific examples of the structure of the organic group represented by X in the general formula (3) also include divalent groups represented by the following general formula (4) and the following general formula (5):

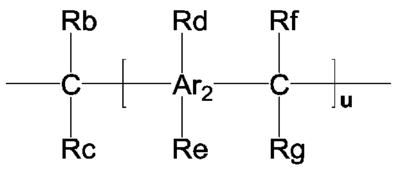

(4)

wherein each $Ar_2$ independently represents a phenylene group, a naphthylene group, or a biphenylene group; when u is 2 or larger, the $Ar_2$ groups may be the same as or different from each other; Rb, Rc, Rf, and Rg each independently represent a hydrogen atom, an alkyl group having 1 to 6 carbon atoms, an aryl group having 6 to 12 carbon atoms, a trifluoromethyl group, or an aryl group having at least one phenolic hydroxy group; Rd and Re each independently represent a hydrogen atom, an alkyl group having 1 to 6 carbon atoms, an aryl group having 6 to 12 carbon atoms, an alkoxy group having 1 to 4 carbon atoms, or a hydroxy group; and u represents an integer of 0 to 5.

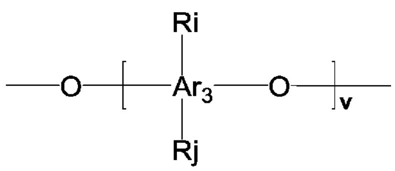

(5)

wherein each $Ar_3$ independently represents a phenylene group, a naphthylene group, or a biphenylene group; a plurality of $Ar_3$ groups may be the same as or different from each other; Ri and Rj each independently represent a hydrogen atom, an alkyl group having 1 to 6 carbon atoms, an aryl group having 6 to 12 carbon atoms, a benzyl group, an alkoxy group having 1 to 4 carbon atoms, a hydroxy group, a trifluoromethyl group, or an aryl group substituted by at least one cyanato group; and

v represents an integer of 0 to 5 (the compound may be a mixture of compounds differing in v).

Further examples of X in the general formula (3) include a divalent group represented by the following formula:

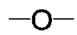 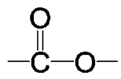 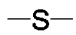 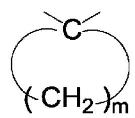 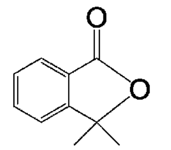

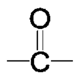 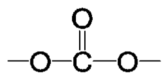 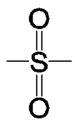 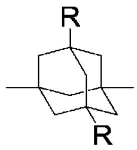 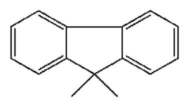

wherein m represents an integer of 4 to 7; and each R independently represents a hydrogen atom or an alkyl group having 1 to 6 carbon atoms.

Specific examples of $Ar_2$ in the general formula (4) and $Ar_3$ in the general formula (5) include, a 1,4-phenylene group, a 1,3-phenylene group, a 4,4'-biphenylene group, a 2,4'-biphenylene group, a 2,2'-biphenylene group, a 2,3'-biphenylene group, a 3,3'-biphenylene group, a 3,4'-biphenylene group, a 2,6-naphthylene group, a 1,5-naphthylene group, a 1,6-naphthylene group, a 1,8-naphthylene group, a 1,3-naphthylene group, a 1,4-naphthylene group, and a 2,7-naphthylene group.

The alkyl group and the aryl group represented by Rb to Rg in the general formula (4) and Ri and Rj in the general formula (5) are as defined in the general formula (3).

Specific examples of the cyanato-substituted aromatic compound represented by the general formula (3) include, but are not particularly limited to, cyanatobenzene, 1-cyanato-2-, 1-cyanato-3-, or 1-cyanato-4-methylbenzene, 1-cyanato-2-, 1-cyanato-3-, or 1-cyanato-4-methoxybenzene, 1-cyanato-2,3-, 1-cyanato-2,4-, 1-cyanato-2,5-, 1-cyanato-2,6-, 1-cyanato-3,4- or 1-cyanato-3,5-dimethylbenzene, cyanatoethylbenzene, cyanatobutylbenzene, cyanatooctylbenzene, cyanatononylbenzene, 2-(4-cyanatophenyl)-2-phenylpropane (4-α-cumylphenol cyanate), 1-cyanato-4-cyclohexylbenzene, 1-cyanato-4-vinylbenzene, 1-cyanato-2- or 1-cyanato-3-chlorobenzene, 1-cyanato-2,6-dichlorobenzene, 1-cyanato-2-methyl-3-chlorobenzene, cyanatonitrobenzene, 1-cyanato-4-nitro-2-ethylbenzene, 1-cyanato-2-methoxy-4-allylbenzene (eugenol cyanate), methyl(4-cyanatophenyl)sulfide, 1-cyanato-3-trifluoromethylbenzene, 4-cyanatobiphenyl, 1-cyanato-2- or 1-cyanato-4-acetylbenzene, 4-cyanatobenzaldehyde, 4-cyanatobenzoic acid methyl ester, 4-cyanatobenzoic acid phenyl ester, 1-cyanato-4-acetaminobenzene, 4-cyanatobenzophenone, 1-cyanato-2,6-di-tert-butylbenzene, 1,2-dicyanatobenzene, 1,3-dicyanatobenzene, 1,4-dicyanatobenzene, 1,4-dicyanato-2-tert-butylbenzene, 1,4-dicyanato-2,4-dimethylbenzene, 1,4-dicyanato-2,3,4-trimethylbenzene, 1,3-dicyanato-2,4,6-trimethylbenzene, 1,3-dicyanato-5-methylbenzene, 1-cyanato or 2-cyanatonaphthalene, 1-cyanato-4-methoxynaphthalene, 2-cyanato-6-methylnaphthalene, 2-cyanato-7-methoxynaphthalene, 2,2'-dicyanato-1,1'-binaphthyl, 1,3-, 1,4-, 1,5-, 1,6-, 1,7-, 2,3-, 2,6- or 2,7-dicyanatonaphthalene, 2,2'- or 4,4'-dicyanatobiphenyl, 4,4'-dicyanatooctafluorobiphenyl, 2,4'- or 4,4'-dicyanatodiphenylmethane, bis(4-cyanato-3,5-dimethylphenyl)methane, 1,1-bis(4-cyanatophenyl)ethane, 1,1-bis(4-cyanatophenyl)propane, 2,2-bis(4-cyanatophenyl)propane, 2,2-bis(4-cyanato-3-methylphenyl)propane, 2,2-bis(2-cyanato-5-biphenylyl)propane, 2,2-bis(4-cyanatophenyl)hexafluoropropane, 2,2-bis(4-cyanato-3,5-dimethylphenyl)propane, 1,1-bis(4-cyanatophenyl)butane, 1,1-bis(4-cyanatophenyl)isobutane, 1,1-bis(4-cyanatophenyl) pentane, 1,1-bis(4-cyanatophenyl)-3-methylbutane, 1,1-bis(4-cyanatophenyl)-2-methylbutane, 1,1-bis(4-cyanatophenyl)-2,2-dimethylpropane, 2,2-bis(4-cyanatophenyl)butane, 2,2-bis(4-cyanatophenyl)pentane, 2,2-bis(4-cyanatophenyl)hexane, 2,2-bis(4-cyanatophenyl)-3-methylbutane, 2,2-bis(4-cyanatophenyl)-4-methylpentane, 2,2-bis(4-cyanatophenyl)-3,3-dimethylbutane, 3,3-bis(4-cyanatophenyl) hexane, 3,3-bis(4-cyanatophenyl)heptane, 3,3-bis(4-cyanatophenyl)octane, 3,3-bis(4-cyanatophenyl)-2-methylpentane, 3,3-bis(4-cyanatophenyl)-2-methylhexane, 3,3-bis(4-cyanatophenyl)-2,2-dimethylpentane, 4,4-bis(4-cyanatophenyl)-3-methylheptane, 3,3-bis(4-cyanatophenyl)-2-methylheptane, 3,3-bis(4-cyanatophenyl)-2,2-dimethylhexane, 3,3-bis(4-cyanatophenyl)-2,4-dimethylhexane, 3,3-bis(4-cyanatophenyl)-2,2,4-trimethylpentane, 2,2-bis(4-cyanatophenyl)-1,1,1,3,3,3-hexafluoropropane, bis(4-cyanatophenyl)phenylmethane, 1,1-bis(4-cyanatophenyl)-1-phenylethane, bis(4-cyanatophenyl)biphenylmethane, 1,1-bis(4-cyanatophenyl)cyclopentane, 1,1-bis(4-cyanatophenyl)cyclohexane, 2,2-bis(4-cyanato-3-isopropylphenyl)propane, 1,1-bis(3-cyclohexyl-4-cyanatophenyl)cyclohexane, bis(4-cyanatophenyl)diphenylmethane, bis(4-cyanatophenyl)-2,2-dichloroethylene, 1,3-bis[2-(4-cyanatophenyl)-2-propyl]benzene, 1,4-bis[2-(4-cyanatophenyl)-2-propyl]benzene, 1,1-bis(4-cyanatophenyl)-3,3,5-trimethylcyclohexane, 4-[bis(4-cyanatophenyl)methyl]biphenyl, 4,4-dicyanatobenzophenone, 1,3-bis(4-cyanatophenyl)-2-propen-1-one, bis(4-cyanatophenyl) ether, bis(4-cyanatophenyl)sulfide, bis(4-cyanatophenyl)sulfone, 4-cyanatobenzoic acid-4-cyanatophenyl ester (4-cyanatophenyl-4-cyanatobenzoate), bis-(4-cyanatophenyl)carbonate, 1,3-bis(4-cyanatophenyl)adamantane, 1,3-bis(4-cyanatophenyl)-5,7-dimethyladamantane, 1,3-bis(3-methyl-4-cyanatophenyl)-5,7-dimethyladamantane, 3,3-bis(4-cyanatophenyl)isobenzofuran-1(3H)-one (phenolphthalein cyanate), 3,3-bis(4-cyanato-3-methylphenyl)isobenzofuran-1(3H)-one (o-cresol phthalein cyanate), 9,9'-bis(4-cyanatophenyl)fluorene, 9,9-bis(4-cyanato-3-methylphenyl)fluorene, 9,9-bis(2-cyanato-5-biphenylyl)fluorene, tris(4-cyanatophenyl)methane, 1,1,1-tris(4-cyanatophenyl)ethane, 1,1,3-tris(4-cyanatophenyl)propane, $\alpha,\alpha,\alpha'$-tris(4-cyanatophenyl)-1-ethyl-4-isopropylbenzene, 1,1,2,2-tetrakis(4-cyanatophenyl)ethane, tetrakis(4-cyanatophenyl)methane, 2,4,6-tris(N-methyl-4-cyanatoanilino)-1,3,5-triazine, 2,4-bis(N-methyl-4-cyanatoanilino)-6-(N-methylanilino)-1,3,5-triazine, bis(N-4-cyanato-2-methylphenyl)-4,4'-oxydiphthalimide, bis(N-3-cyanato-4-methylphenyl)-4,4'-oxydiphthalimide, bis(N-4-cyanatophenyl)-4,4'-oxydiphthalimide, bis(N-4-cyanato-2-methylphenyl)-4,4'-(hexafluoroisopropylidene)diphthalimide, tris(3,5-dimethyl-4-cyanatobenzyl)isocyanurate, 2-phenyl-3,3-bis(4-cyanatophenyl)phthalimidine, 2-(4-methylphenyl)-3,3-bis(4-cyanatophenyl)phthalimidine, 2-phenyl-3,3-bis(4-cyanato-3-methylphenyl)phthalimidine, 1-methyl-3,3-bis(4-cyanatophenyl)indolin-2-one, 2-phenyl-3,3-bis(4-cyanatophenyl)indolin-2-one, cyanation products obtained in the same way as above from phenol novolac resins and cresol novolac resins (resins obtained by reacting phenol, alkyl-substituted phenol, or halogen-substituted phenol with a formaldehyde compound such as formalin or paraformaldehyde in an acidic solution by a method known in the art), trisphenol novolac resins (resins obtained by reacting hydroxybenzaldehyde with phenol in the presence of an acidic catalyst), fluorene novolac resins (resins obtained by reacting a fluorenone compound with a 9,9-bis(hydroxyaryl)fluorene in the presence of an acidic catalyst), phenol aralkyl resins, cresol aralkyl resins, naphthol aralkyl resins, and biphenyl aralkyl resins (resins obtained by reacting a bishalogenomethyl compound represented by $Ar_2$-$(CH_2Y)_2$ ($Ar_2$ represents a phenylene group, a naphthylene group, or a biphenylene group, and Y represents a halogen atom; the same holds true for this paragraph) with a phenol compound in the presence or absence of an acidic catalyst, resins obtained by reacting a bis(alkoxymethyl) compound represented by $Ar_2$-$(CH_2OR)_2$ or a bis(hydroxymethyl) compound represented by $Ar_2$-$(CH_2OH)_2$ with a phenol compound in the presence of an acidic catalyst, or resins obtained by polycondensing an aromatic aldehyde compound, an aralkyl compound, and a phenol compound, by a method known in the art), phenol-modified xylene formaldehyde resins (resins obtained by reacting a xylene formaldehyde resin with a phenol compound in the presence of an acidic catalyst by a method known in the art), modified naphthalene formaldehyde resins (resins obtained by reacting a naphthalene formaldehyde resin with a hydroxy-substituted aromatic compound in the presence of an acidic catalyst by a method known in the art), and phenol resins such as phenol-modified dicyclopentadiene resins and phenol resins having a polynaphthylene ether structure (resins obtained by dehydratively condensing a polyvalent hydroxynaphthalene compound having two or more phenolic hydroxy groups in one molecule in the presence of a basic catalyst by a method known in the art), and prepolymers thereof. Among these compounds, a phenol novolac-based cyanic acid ester compound, a naphthol aralkyl-based cyanic acid ester compound, a biphenyl aralkyl-based cyanic acid ester compound, a naphthylene ether-based cyanic acid ester compound, a xylene resin-based cyanic acid ester compound, and an adamantane skeleton-based cyanic acid ester compound are preferred, and a naphthol aralkyl-based cyanic acid ester compound is particularly preferred.

One of or a mixture of two or more of these additional cyanic acid ester compounds can be used.

The method for producing such an additional cyanic acid ester compound is not particularly limited, and a method known in the art can be used. Examples of such a process include a method which involves obtaining or synthesizing a hydroxy group-containing compound having the desired skeleton, and modifying the hydroxy group by an approach known in the art for cyanation. Examples of the approach for cyanating the hydroxy group include an approach described in Ian Hamerton, "Chemistry and Technology of Cyanate Ester Resins", Blackie Academic & Professional.

A resin cured product containing these cyanic acid ester compounds has excellent properties such as a glass transition temperature, low thermal expansibility, and plating adhesion.

In the resin composition of the present embodiment, a content of the cyanic acid ester compound is not particularly limited and is preferably 1 part by mass to 99 parts by mass, more preferably 10 parts by mass to 90 parts by mass, further preferably 20 to 80 parts by mass, still further preferably 30 to 70 parts by mass, even further preferably 40 to 60 parts by mass, with respect to 100 parts by mass of a resin solid in the resin composition from the viewpoint of plating adhesion and heat resistance.

<Maleimide compound>

A compound generally known in the art can be used as the maleimide compound as long as the compound has one or more maleimide groups in one molecule. Examples of the maleimide compound include, but are not particularly limited to, N-phenylmaleimide, N-hydroxyphenylmaleimide, bis(4-maleimidophenyl)methane, 2,2-bis{4-(4-maleimidophenoxy)-phenyl}propane, 4,4-diphenylmethane bismaleimide, bis(3,5-dimethyl-4-maleimidophenyl)methane, bis(3-ethyl-5-methyl-4-maleimidophenyl)methane, bis(3,5-diethyl-4-maleimidophenyl)methane, phenylmethanemaleimide, 3,3-dimethyl-5,5-diethyl-4,4-diphenylmethane bismaleimide, 1,6-bismaleimide-(2,2,4-trimethyl)hexane, o-phenylene bismaleimide, m-phenylene bismaleimide, p-phenylene bismaleimide, o-phenylene biscitraconimide, m-phenylene biscitraconimide, p-phenylene biscitraconimide, 4,4'-diphenylmethane bismaleimide, bis(3,5-dimethyl-4-maleimidophenyl)methane, bis(3-ethyl-5-methyl-4-maleimidophenyl)methane, bis(3,5-diethyl-4-maleimidophenyl)methane, 2,2'-bis [4-(4-maleimidophenoxy)phenyl]propane, 4-methyl-1,3-phenylene bismaleimide, 1,6'-bismaleimido-(2,2,4-trimethyl) hexane, 4,4-diphenyl ether bismaleimide, 4,4-diphenylsulfone bismaleimide, 1,3-bis(3-maleimidophenoxy)benzene, 1,3-bis(4-maleimidophenoxy)benzene, 4,4'-diphenylmethane biscitraconimide, 2,2'-bis[4-(4-citraconimidophenoxy) phenyl]propane, bis(3,5-dimethyl-4-citraconimidophenyl)methane, bis(3-ethyl-5-methyl-4-citraconimidophenyl) methane, bis(3,5-diethyl-4-citraconimidophenyl)methane, polyphenylmethanemaleimide, novolac-based maleimide, biphenyl aralkyl-based maleimide, a maleimide compound represented by the formula (7) given below, a maleimide compound represented by the formula (8) given below, and prepolymers of these maleimide compounds, and prepolymers of these maleimide compounds and amine compounds.

Among these maleimide compounds, novolac-based maleimide and biphenyl aralkyl-based maleimide are particularly preferred. A maleimide compound represented by the formula (6) given below and a maleimide compound represented by the formula (7) given below are preferred, and a maleimide compound represented by the formula (6) given below is more preferred, from the viewpoint of obtaining favorable film performance and being excellent in heat resistance. A commercially available product may be used as the maleimide compound represented by the formula (6) given below. Examples thereof include BMI-2300 (manufactured by Daiwa Fine Chemicals Co., Ltd.). A commercially available product may be used as the maleimide compound represented by the formula (7) given below. Examples thereof include MIR-3000 (manufactured by Nippon Kayaku Co., Ltd.).

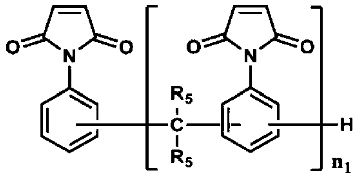

(6)

wherein a plurality of $R_5$ groups each independently represent a hydrogen atom or a methyl group, and $n_1$ represents an integer of 1 or larger, preferably an integer of 1 to 10, more preferably an integer of 1 to 5.

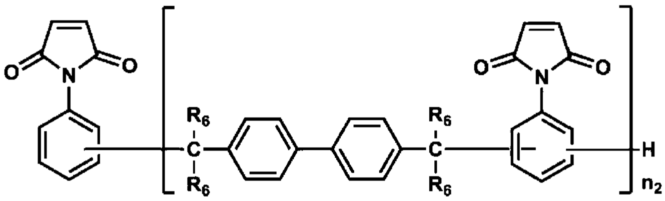

(7)

wherein a plurality of $R_6$ groups each independently represent a hydrogen atom or a methyl group, and $n_2$ represents an integer of 1 or larger, preferably an integer of 1 to 5.

One of or a mixture of two or more of these maleimide compounds can be used.

In the resin composition of the present embodiment, a content of the maleimide compound can be appropriately set according to the desired properties, and is not particularly limited. The content is preferably 1 part by mass to 99 parts by mass, more preferably 10 parts by mass to 90 parts by mass, further preferably 20 parts by mass to 80 parts by mass, still further preferably 25 parts by mass to 75 parts by mass, even further preferably 30 parts by mass to 70 parts by mass, most preferably 40 parts by mass to 60 parts by mass, with respect to 100 parts by mass of a resin solid in the resin composition. When the content falls within the range of 1 to 90 parts by mass, the resulting resin composition is excellent in glass transition temperature and heat resistance of a cured product.

<Phenol resin>

The phenol resin is preferably a phenol resin having two or more hydroxy groups in one molecule, and a phenol resin generally known in the art can be used. Examples of the phenol resin include, but are not particularly limited to, bisphenol A-based phenol resins, bisphenol E-based phenol resins, bisphenol F-based phenol resins, bisphenol S-based phenol resins, phenol novolac resins, bisphenol A novolac-based phenol resins, glycidyl esterbased phenol resins, aralkyl novolac-based phenol resins, biphenyl aralkyl-based phenol resins, cresol novolac-based phenol resins, polyfunctional phenol resins, naphthol resins, naphthol novolac resins, polyfunctional naphthol resins, anthracene-based phenol resins, naphthalene skeleton-modified novolac-based phenol resins, phenol aralkyl-based phenol resins, naphthol aralkyl-based phenol resins, dicyclopentadiene-based phenol resins, biphenyl-based phenol resins, alicyclic phenol resins, polyol-based phenol resins, phosphorus-containing phenol resins, polymerizable unsaturated hydrocarbon group-containing phenol resins, and hydroxy group-containing silicone resins. Among these phenol resins, a biphenyl aralkyl-based phenol

resin, a naphthol aralkyl-based phenol resin, a phosphorus-containing phenol resin, and a hydroxy group-containing silicone resin are preferred from the viewpoint of flame retardance. One of these phenol resins can be used alone, or two or more thereof can be used in combination.

In the resin composition of the present embodiment, a content of the phenol resin is not particularly limited and is preferably 1 part by mass to 99 parts by mass, more preferably 10 parts by mass to 90 parts by mass, with respect to 100 parts by mass of a resin solid in the resin composition. When the content of the phenol resin falls within the range described above, the heat resistance tends to be further improved.

<Epoxy resin>

A compound generally known in the art can be used as the epoxy resin as long as the compound has two or more epoxy groups in one molecule. The epoxy resin is not particularly limited by its type. Examples of the epoxy resin include bisphenol A-based epoxy resins, bisphenol E-based epoxy resins, bisphenol F-based epoxy resins, bisphenol S-based epoxy resins, phenol novolac-based epoxy resins, bisphenol A novolac-based epoxy resins, naphthylene ether-based epoxy resins, xylene novolac-based epoxy resins, dicyclopentadiene novolac-based epoxy resins, biphenyl novolac-based epoxy resins, polyfunctional phenol-based epoxy resins, naphthalene-based epoxy resins, naphthalene skeleton-modified novolac-based epoxy resins, phenol aralkyl-based epoxy resins, dicyclopentadiene novolac-based epoxy resins, biphenyl-based epoxy resins, phenol novolac-based epoxy resins, cresol novolac-based epoxy resins, xylene novolac-based epoxy resins, naphthalene-based epoxy resins, anthracene-based epoxy resins, trifunctional phenol-based epoxy resins, tetrafunctional phenol-based epoxy resins, triglycidyl isocyanurate, glycidyl esterbased epoxy resins, alicyclic epoxy resins, dicyclopentadiene novolac-based epoxy resins, biphenyl novolac-based epoxy resins, phenol aralkyl novolac-based epoxy resins, naphthol aralkyl novolac-based epoxy resins, aralkyl novolac-based epoxy resins, biphenyl aralkyl-based epoxy resins, naphthol aralkyl-based epoxy resins, dicyclopentadiene-based epoxy resins, polyol-based epoxy resins, phosphorus-containing epoxy resins, compounds having an epoxidized double bond such as glycidylamine, glycidyl ester, and butadiene, and compounds obtained through the reaction of hydroxy group-containing silicone resins with epichlorohydrin, , and halides thereof. Among these epoxy resins, a biphenyl aralkyl-based epoxy resin, a naphthylene ether-based epoxy resin, a polyfunctional phenol-based epoxy resin, and a naphthalene-based epoxy resin are preferred from the viewpoint of flame retardance and heat resistance.

One of or a mixture of two or more of these epoxy resins can be used.

A content of the epoxy resin according to the present embodiment can be appropriately set according to the desired properties, and is not particularly limited. The content is preferably 1 part by mass to 99 parts by mass, more preferably 10 parts by mass to 90 parts by mass, further preferably 20 parts by mass to 80 parts by mass, still further preferably 25 parts by mass to 75 parts by mass, even further preferably 30 parts by mass to 70 parts by mass, most preferably 40 parts by mass to 60 parts by mass, with respect to 100 parts by mass of a resin solid in the resin composition. When the content of the phenol resin falls within the range described above, the heat resistance tends to be further improved.

<Oxetane resin>

An oxetane resin generally known in the art can be used as the oxetane resin. Examples of the oxetane resin include: oxetane; alkyloxetanes such as 2-methyloxetane, 2,2-dimethyloxetane, 3-methyloxetane, and 3,3-dimethyloxetane; and 3-methyl-3-methoxymethyloxetane, 3,3'-di(trifluoromethyl)perfluorooxetane, 2-chloromethyloxetane, biphenyl-based oxetane, and 3,3-bis(chloromethyl)oxetane. Examples of commercially available products include OXT-101 (trade name, manufactured by Toagosei Co., Ltd.) and OXT-121 (trade name, manufactured by Toagosei Co., Ltd.).

One of or a mixture of two or more of these oxetane resins can be used.

In the resin composition of the present embodiment, a content of the oxetane resin is not particularly limited and is preferably 1 part by mass to 99 parts by mass, more preferably 10 parts by mass to 90 parts by mass, with respect to 100 parts by mass of a resin solid in the resin composition. When the content of the oxetane resin falls within the range described above, the heat resistance tends to be further improved.

<Benzoxazine compound>

The benzoxazine compound is preferably a compound having two or more dihydrobenzoxazine rings in one molecule, and a compound generally known in the art can be used. Examples of the benzoxazine compound include bisphenol A-based benzoxazine BA-BXZ (trade name, manufactured by Konishi Chemical Ind. Co., Ltd.), bisphenol F-based benzoxazine BF-BXZ (trade name, manufactured by Konishi Chemical Ind. Co., Ltd.), bisphenol S-based benzoxazine BS-BXZ (trade name, manufactured by Konishi Chemical Ind. Co., Ltd.), P-d-based benzoxazine (trade name, manufactured by Shikoku Chemicals Corp.), F-a-based benzoxazine (trade name, manufactured by Shikoku

Chemicals Corp.), and phenolphthalein-based benzoxazine.

One of or a mixture of two or more of these benzoxazine compounds can be used.

In the resin composition of the present embodiment, a content of the benzoxazine compound is not particularly limited and is preferably 1 part by mass to 99 parts by mass, more preferably 10 parts by mass to 90 parts by mass, with respect to 100 parts by mass of a resin solid in the resin composition. When the content of the benzoxazine compound falls within the range described above, the heat resistance tends to be further improved.

<Compound having polymerizable unsaturated group>

A compound generally known in the art can be used as the compound having a polymerizable unsaturated group. Examples of the compound having a polymerizable unsaturated group include: vinyl compounds such as ethylene, propylene, styrene, divinylbenzene, and divinylbiphenyl, monohydric or polyhydric alcohol (meth)acrylates such as methyl (meth)acrylate, 2-hydroxyethyl (meth)acrylate, 2-hydroxypropyl (meth)acrylate, polypropylene glycol di(meth) acrylate, trimethylolpropane di(meth)acrylate, trimethylolpropane tri(meth)acrylate, pentaerythritol tetra(meth)acrylate, and dipentaerythritol hexa(meth)acrylate, epoxy (meth)acrylates such as bisphenol A-based epoxy (meth)acrylate and bisphenol F-based epoxy (meth)acrylate, and benzocyclobutene resins.

One of or a mixture of two or more of these compounds having a polymerizable unsaturated group can be used.

In the resin composition of the present embodiment, a content of the compound having a polymerizable unsaturated group is not particularly limited and is preferably 1 part by mass to 99 parts by mass, more preferably 10 parts by mass to 90 parts by mass, with respect to 100 parts by mass of a resin solid in the resin composition from the viewpoint of improving developability and heat resistance.

<Polymerization catalyst>

The resin composition according to the present embodiment can be further supplemented with a compound acting as a polymerization catalyst for the cyanic acid ester compound, the maleimide compound, the phenol resin, the epoxy resin, the oxetane resin, and the compound having a polymerizable unsaturated group, in addition to the compounds and the resins described above. Examples of the polymerization catalyst include: metal salts such as zinc octanoate, zinc naphthenate, cobalt naphthenate, copper naphthenate, and acetylacetone iron; phenol compounds such as octylphenol and nonylphenol; alcohols such as 1-butanol and 2-ethylhexanol; imidazole derivatives such as 2-methylimidazole, 2-ethyl-4-methylimidazole, 2-phenylimidazole, 1-cyanoethyl-2-phenylimidazole, 1-cyanoethyl-2-ethyl-4-methylimidazole, 2-phenyl-4,5-dihydroxymethylimidazole, and 2-phenyl-4-methyl-5-hydroxymethylimidazole; amine compounds such as dicyandiamide, benzyldimethylamine, and 4-methyl-N,N-dimethylbenzylamine; and phosphorus compounds of phosphines or phosphoniums. Alternatively, an epoxyimidazole adduct compounds, a peroxide such as benzoyl peroxide, p-chlorobenzoyl peroxide, di-t-butyl peroxide, diisopropyl peroxycarbonate, or di-2-ethylhexyl peroxycarbonate, or an azo compound such as azobisisobutyronitrile may be used as the polymerization catalyst. Commercially available products of these polymerization catalysts may be used. Examples of the commercially available products include Amicure PN-23 (trade name, manufactured by Ajinomoto Fine-Techno Co., Inc.), Novacure HX-3721 (trade name, manufactured by Asahi Kasei Corp.), and Fujicure FX-1000 (trade name, manufactured by Fuji Kasei Kogyo Co., Ltd.).

One of or a mixture of two or more of these polymerization catalysts can be used.

In the resin composition of the present embodiment, a content of the polymerization catalyst is not particularly limited and is usually 0.1 to 10 parts by mass with respect to 100 parts by mass of the resin composition in the resin composition.

<Filler>

An inorganic filler can be used in the resin composition according to the present embodiment and is not particularly limited by its type. Examples of inorganic fillers include inorganic fillers including: talc, clay, baked clay, unbaked clay, mica, kaolin, baked kaolin, baked talc, E glass, A glass, NE glass, C glass, L glass, D glass, S glass, M glass G20, short glass fiber (including fine glass powders such as E glass, T glass, D glass, S glass, and Q glass), hollow glass, and spherical glass; silicas such as natural silica, molten silica, synthetic silica, amorphous silica, Aerosil, and hollow silica; silicate and white carbon; oxides such as titanium white, titanium oxide, alumina, gibbsite, boehmite, zinc oxide, magnesium oxide, zirconium oxide, and molybdenum oxide; molybdenum compounds such as molybdenum oxide and zinc molybdate; carbonates such as calcium carbonate, magnesium carbonate, and hydrotalcite; metal hydroxides such as aluminum hydroxide, heat-treated products of aluminum hydroxide (which is obtained by heat-treating aluminum hydroxide and removing a portion of crystallization water), magnesium hydroxide, and calcium hydroxide; sulfates or sulfites such as barium sulfate, calcium sulfate, and calcium sulfite; borates such as zinc borate, barium metaborate,

aluminum borate, calcium borate, and sodium borate; nitrides such as aluminum nitride, boron nitride, boron nitride agglomerates, silicon nitride, and carbon nitride; carbides such as silicon carbide; titanates such as strontium titanate and barium titanate; and zinc stannate, zinc molybdate, silicone powders, silicone composite powders, and silicone resin powders. Examples of organic fillers include styrene-based, butadiene-based, acrylic, fluorine-based, urethane-based, and polyethylene-based rubber powders, styrene-butadiene rubber, core-shell rubber powders, silicone resin powders, silicon rubber powders, and silicone composite powders.

Among these fillers, silica, boehmite, talc, mica, glass, aluminum nitride, boron nitride, a silicone powder, a silicone composite powder, a molybdenum compound, and a rubber powder are preferred.

Particularly, silica is preferred, and molten silica is particularly preferred, from the viewpoint of improving the heat resistance of a cured product and obtaining favorable film performance. Specific examples of the silica include SFP-130MC manufactured by Denka Co., Ltd. and SC2050-MB, SC1050-MLE, YA010C-MFN, and YA050C-MJA manufactured by Admatechs Co., Ltd.

One of these fillers can be used alone, or two or more thereof can be used as a mixture. These fillers (I) differing in shape (spherical or crushed) or size can be mixed and used at an increased content.

In the resin composition of the present embodiment, a content of the filler can be appropriately set according to the desired properties, and is not particularly limited. The content is preferably 50 to 1600 parts by mass, more preferably 60 to 1000 parts by mass, further preferably 70 to 500 parts by mass, particularly preferably 80 to 200 parts by mass, most preferably 80 to 150 parts by mass, with respect to 100 parts by mass of a resin solid in the resin composition. When the content of the filler falls within the range described above, the resulting resin composition tends to have favorable moldability and be excellent in the flame retardance, heat resistance, and moisture-absorbing heat resistance of a cured product of the resin composition containing the filler.

The inorganic filler may be further surface-treated in advance with a treating agent. At least one or more types of compounds selected from the group consisting of functional group-containing silanes, cyclic oligosiloxanes, organohalosilanes, and alkylsilazanes can be suitably used as the treating agent. Among these treating agents, the surface treatment of spherical silica using organohalosilanes and alkylsilazanes is suitable for hydrophobizing silica surface and is preferred from the viewpoint of the excellent dispersibility of the spherical silica in the resin composition.

The inorganic filler is preferably used in combination with a silane coupling agent or a wetting dispersant.

A silane coupling agent generally used in the surface treatment of inorganic matter can be suitably used and is not particularly limited by its type. Specific examples of the silane coupling agent include: aminosilane coupling agents such as γ-aminopropyltriethoxysilane and N-β-(aminoethyl)-γ-aminopropyltrimethoxysilane; epoxysilane coupling agents such as γ-glycidoxypropyltrimethoxysilane and β-(3,4-epoxycyclohexyl)ethyltrimethoxysilane; vinylsilane coupling agents such as γ-methacryloxypropyltrimethoxysilane and vinyl-tri(β-methoxyethoxy)silane; cationic silane coupling agents such as N-β-(N-vinylbenzylaminoethyl)-γ-aminopropyltrimethoxysilane hydrochloride; and phenylsilane coupling agents. One of these silane coupling agents can be used alone, or two or more thereof can be used in appropriate combination.

A wetting dispersant generally used for a coating material can be suitably used and is not particularly limited by its type. Use of the wetting dispersant can improve the dispersibility of the filler and the adhesion strength between a polymer and/or a resin and the filler. A copolymer-based wetting dispersant is preferably used as the wetting dispersant, and a commercially available product may be used. Specific examples of the commercially available product include Disperbyk(R)-110, -111, -161, and -180, BYK(R)-W996, BYK(R)-W9010, BYK(R)-W903, and BYK(R)-W940 (all are trade names) manufactured by BYK Japan K.K. One of these wetting dispersants can be used alone, or two or more thereof can be used in appropriate combination.

In the resin composition according to the present embodiment, a content of the wetting dispersant is not particularly limited and is usually 0.1 to 10 parts by mass with respect to 100 parts by mass of the resin composition.

<Curing accelerator>

The resin composition of the present embodiment may optionally contain a curing accelerator for appropriately adjusting a curing rate.

A curing accelerator generally used for cyanic acid ester compounds, epoxy resins, or the like can be suitably used as this curing accelerator and is not particularly limited by its type. Specific examples of the curing accelerator include: organometal salts such as zinc octanoate, zinc naphthenate, cobalt naphthenate, copper naphthenate, acetylacetone iron, nickel octanoate, and manganese octanoate; phenol compounds such as phenol, xylenol, cresol, resorcin, catechol, octylphenol, and nonylphenol; alcohols such as 1-butanol and 2-ethylhexanol; imidazoles such as 2-methylimidazole, 2-ethyl-4-methylimidazole, 2-phenylimidazole, 1-cyanoethyl-2-phenylimidazole, 1-cyanoethyl-2-ethyl-4-methylimidazole, 2-phenyl-4,5-dihydroxymethylimidazole, and 2-phenyl-4-methyl-5-hydroxymethylimidazole, and derivatives of these imidazoles, such as adducts of carboxylic acids or acid anhydrides thereof; amine compounds such as dicyandiamide, benzyldimethylamine, and 4-methyl-N,N-dimethylbenzylamine; phosphorus compounds such as phosphine compounds, phosphine oxide compounds, phosphonium compounds, and diphosphine compounds; epoxyimidazole adduct compounds; peroxides such as benzoyl peroxide, p-chlorobenzoyl peroxide, di-t-butyl peroxide, diisopropyl peroxycarbonate, and di-2-ethylhexyl peroxycarbonate; azo compounds such as azobisisobutyronitrile; and N,N-dimethylaminopyridine.

A commercially available product may be used as the curing accelerator. Examples of the commercially available product include Amicure(R) PN-23 (trade name, manufactured by Ajinomoto Fine-Techno Co., Inc.), Novacure(R) HX-3721 (trade name, manufactured by Asahi Kasei Corp.), and Fujicure(R) FX-1000 (trade name, manufactured by Fuji Kasei Kogyo Co., Ltd.). One of these curing accelerators can be used alone, or two or more thereof can be used in appropriate combination.

The amount of the curing accelerator used can be appropriately adjusted in consideration of the degree of cure of resins, the viscosity of the resin composition, etc., and is not particularly limited. The amount of the curing accelerator used is usually approximately 0.005 to 10 parts by mass with respect to 100 parts by mass of a resin solid in the resin composition.

<Solvent>

The resin composition according to the present embodiment may optionally comprise a solvent. In this case, the resin composition can be used in a form in which at least a portion, preferably the whole of each component mentioned above is dissolved or compatibilized in an organic solvent (solution or varnish). The solvent is not particularly limited as long as at least a portion, preferably the whole of each component mentioned above can be dissolved or compatibilized therein. Examples thereof include: ketone solvents such as acetone, methyl ethyl ketone, methyl isobutyl ketone, and cyclohexanone; cellosolve solvents such as propylene glycol monomethyl ether and propylene glycol monomethyl ether acetate; ester solvents such as methyl lactate, methyl acetate, ethyl acetate, butyl acetate, isoamyl acetate, ethyl lactate, methyl methoxypropionate, and methyl hydroxyisobutyrate; alcohol solvents such as methanol, ethanol, isopropanol, and 1-ethoxy-2-propanol; amides such as dimethylacetamide and dimethylformamide; and aromatic hydrocarbons such as toluene, xylene, and anisole.

One of these solvents can be used alone, or two or more thereof can be used as a mixture.

<Other components>

In addition, the resin composition of the present embodiment may optionally contain additives known in the art including: various polymer compounds such as other thermoplastic resins, thermoplastic resins and oligomers thereof, and elastomers, except for those listed above; and flame retardant compounds, curing catalysts, curing accelerators, coloring pigments, antifoaming agents, surface adjusters, flame retardants, ultraviolet absorbers, antioxidants, photopolymerization initiators, fluorescent whitening agents, photosensitizers, dyes, pigments, thickeners, lubricants, flow modifiers, dispersants, leveling agents, brightening agents, surface conditioners, and polymerization inhibitors, except for those listed above. Examples of the flame retardant compounds include: bromine compounds such as 4,4-dibromobiphenyl; phosphoric acid esters including aromatic condensed phosphoric acid ester and halogencontaining condensed phosphoric acid ester; melamine phosphate; phosphorus-containing epoxy resins; nitrogen compounds such as melamine and benzoguanamine; oxazine ring-containing compounds; phosphate compounds of phosphorus compounds; and silicone compounds. The resin composition of the present embodiment may optionally contain a solvent.

One of or a mixture of two or more of these optional additives can be used.

When the resin composition according to the present embodiment contains other components, a content thereof depends on a purpose and is not particularly limited. The content of each component is usually 0.1 to 10 parts by mass with respect to 100 parts by mass of the resin composition.

<Method for producing resin composition>

The resin composition according to the present embodiment can be produced by mixing the components mentioned above by an approach known in the art. Examples of the mixing method include, but are not particularly limited to, methods using a high-speed mixer, a Nauta mixer, a ribbon blender, a kneader, an intensive mixer, a universal mixer, a dissolver, a static mixer, or the like. For the mixing, methods for adding the cyanic acid ester compound, various additives, and a solvent are not particularly limited. The type of the solvent is not particularly limited as long as the solvent is capable of dissolving resins in the resin composition. Specific examples thereof are as mentioned above.

Treatment (stirring, mixing, kneading treatment, etc.) known in the art for uniformly dissolving or dispersing each component can be performed in the preparation of the resin composition. Stirring and dispersing treatment using a stirring vessel equipped with a stirrer having an appropriate stirring ability can be performed for the uniform dispersion of the filler. This enhances the dispersibility of the filler in the resin composition. The stirring, mixing, or kneading treatment can be appropriately performed using an apparatus known in the art such as an apparatus aimed at mixing, for example, a ball mill or a bead mill, or a revolution- or rotation-type mixing apparatus.

The resin composition according to the present embodiment can be cured by heat, light, or the like to obtain a cured product. The cured product can be obtained, for example, by melting the resin composition or dissolving the resin composition in a solvent, and then injecting the resultant into a mold, followed by curing under usual conditions. For heat curing, the curing temperature is preferably in the range of 120°C to 300°C from the viewpoint of further accelerating the curing and further suppressing the degradation of the cured product.

[Purpose of resin composition]

The material for encapsulation of the present embodiment comprises the resin composition and can be produced using the resin composition. A method generally known in the art can be appropriately applied to a method for producing the material for encapsulation and is not particularly limited. For example, the resin composition can be mixed with various additives or solvents, etc. known to be used for the production of a material for encapsulation, using a mixer known in the art to produce the material for encapsulation. For the mixing, a method generally known in the art can be appropriately applied to a method for adding the resin composition, various additives, and solvents and is not particularly limited.

The resin composition of the present embodiment can be used as a prepreg, a metal foil-clad laminate, an insulating layer for printed circuit boards, and a material for semiconductor package configurations. For example, a prepreg can be obtained by impregnating or coating a base material with a solution of the resin composition of the present embodiment dissolved in a solvent, followed by drying.

Also, a resin sheet can be obtained by drying a solution of the resin composition of the present embodiment dissolved in a solvent. The resin sheet can be used as a film for buildup or a dry film solder resist. The resin composition may be used in an uncured state by the mere drying of the solvent or may be used, if necessary, in a semi-cured state (state converted to B-stage).

Hereinafter, the prepreg of the present embodiment will be described in detail. The prepreg of the present embodiment comprises a base material and the resin composition of the present embodiment with which the base material is impregnated or coated. The method for producing the prepreg of the present embodiment is not particularly limited as long as the method is a method for producing a prepreg by combining the resin composition of the present embodiment with the base material. Specifically, the prepreg of the present embodiment can be produced through semi-curing by, for example, a method of impregnating or coating the base material with the resin composition of the present embodiment, followed by drying in a dryer of 120 to 220°C for approximately 2 to 15 minutes. In this respect, the amount of the resin composition attached to the base material, i.e., a content of the resin composition (including the filler) with respect to the total amount of the prepreg after semi-curing, is preferably in the range of 20 to 99% by mass.

The base material for use in the production of the prepreg of the present embodiment may be a base material known in the art for use in various printed circuit board materials. Examples of such a base material include, but are not limited to: glass fibers such as E glass, D glass, L glass, S glass, T glass, Q glass, UN glass, NE glass, and spherical glass; non-glass inorganic fibers such as quartz; organic fibers such as polyimide, polyamide, and polyester; and woven fabrics such as liquid-crystal polyester. For example, woven fabrics, nonwoven fabrics, rovings, chopped strand mats, and surfacing mats are known as shapes of the base material, and any of these shapes may be used. One of these base materials can be used alone, or two or more thereof can be used in appropriate combination. Particularly, a woven fabric treated by ultra-opening or compaction is preferred from the viewpoint of dimensional stability. Alternatively, a glass woven fabric surface-treated with a silane coupling agent such as epoxysilane or aminosilane is preferred from the viewpoint of moisture-absorbing heat resistance. Also, a liquid-crystal polyester woven fabric is preferred from the viewpoint of electrical properties. The thickness of the base material is not particularly limited and is preferably in the range of 0.01 to 0.2 mm for the purpose of laminates.

The prepreg for a structural material of the present embodiment comprises a base material and the resin composition with which the base material is impregnated or coated. The prepreg for a structural material can be produced by impregnating or coating an inorganic and/or an organic fiber base material(s) with the resin composition, followed by further drying, if necessary.

Examples of the base material include, but are not particularly limited to, inorganic fiber base materials including glass fiber base materials such as glass woven fabrics and glass nonwoven fabrics, and organic fiber base materials including: polyamide resin fibers such as polyamide resin fiber, aromatic polyamide resin fiber, and wholly aromatic polyamide resin fiber; polyester resin fibers such as polyester resin fiber, aromatic polyester resin fiber, and wholly aromatic polyester resin fiber; synthetic fiber base materials constituted by woven fabrics or nonwoven fabrics composed mainly of polyimide resin fiber, fluorine resin fiber, or the like; and paper base materials composed mainly of kraft paper, cotton linter paper, mixture paper of linter and kraft pulp, or the like. The base material can be appropriately selected for use from those known in the art according to performance required for the prepreg, for example, strength, the rate of water absorption, and the coefficient of thermal expansion. Examples of the glass constituting the glass fiber base materials include, but are not particularly limited to, E glass, C glass, A glass, S glass, D glass, NE glass, T glass, and H glass.

A method generally known in the art can be appropriately applied to a method for producing the prepreg for a

structural material and is not particularly limited. For example, resin varnish is prepared using the afore-mentioned resin composition, and the prepreg can be produced by the application of a method of dipping the base material in the resin varnish, a method of coating the base material with the resin varnish using various coaters; a method of spraying the resin varnish to the base material using a spray, or the like. Among these methods, the method of dipping the base material in the resin varnish is preferred. The impregnating properties of the resin composition for the base material can thereby be improved. In the case of dipping the base material in the resin varnish, a usual impregnation or coating facility can be used. For example, a method for producing a prepreg through conversion to B-stage by impregnating the inorganic and/or the organic fiber base material(s) with the resin composition varnish, followed by drying can be applied thereto.

The metal foil-clad laminate of the present embodiment comprises at least one or more layers of the prepreg mentioned above, and metal foil disposed on one side or both sides of the prepreg. Specifically, the metal foil-clad laminate can be prepared by preparing one or more layers of the prepreg mentioned above, and disposing metal (e.g., copper or aluminum) foil on one side or both sides thereof, followed by lamination molding. In this context, the metal foil used is not particularly limited as long as the metal foil is used in printed circuit board materials. Copper foil such as rolled copper foil or electrolytic copper foil is preferred. The thickness of the metal foil is not particularly limited and is preferably 2 to 70 μm, more preferably 3 to 35 μm. A usual approach for use in the preparation of laminates and multilayer boards for printed circuit boards can be applied to molding conditions. The metal foil-clad laminate of the present embodiment can be produced by using, for example, a multiplaten press, a multiplaten vacuum press, a continuous molding machine, or an autoclave molding machine and performing lamination molding under conditions involving a temperature of 180 to 350°C, a heating time of 100 to 300 minutes, and a surface pressure of 20 to 100 kg/cm$^2$. Alternatively, the prepreg described above may be lamination-molded in combination with a separately prepared wiring board for an inner layer to obtain a multilayer board. The method for producing the multilayer board involves, for example, disposing 35-μm copper foil on both sides of one prepreg mentioned above, lamination-molding the resultant under the conditions described above, then forming an inner layer circuit, and carrying out the black oxide treatment of the circuit to form an inner layer circuit board. This inner layer circuit board and the prepreg are alternately disposed one by one. Copper foil is further disposed on the outermost layer. The resultant is lamination-molded, preferably in vacuum, under the conditions described above. In this way, the multilayer board can be prepared.

The metal foil-clad laminate of the present embodiment can be suitably used as a printed circuit board by further forming a pattern. The printed circuit board can be produced according to a routine method, and a method for producing the printed circuit board is not particularly limited. Hereinafter, one example of the method for producing the printed circuit board will be described. First, the metal foil-clad laminate mentioned above, such as a copper-clad laminate is prepared. Next, a surface of the metal foil-clad laminate is subjected to etching treatment for the formation of an inner layer circuit to prepare an inner layer substrate. The inner layer circuit surface of this inner layer substrate is surface-treated, if necessary, for enhancing adhesion strength. Subsequently, a required number of layers of the prepreg mentioned above is put on the resulting inner layer circuit surface. Metal foil for an outer layer circuit is laterally laminated thereon, followed by integral molding by heating under increased pressure. In this way, a multilayer laminate is produced in which the insulating layer composed of the base material and a cured product of the thermosetting resin composition is formed between the inner layer circuit and the metal foil for an outer layer circuit. Subsequently, this multilayer laminate is processed by hole drilling for through-holes or via holes. Then, the inside walls of these holes are coated with a metal plating film for the community between the inner layer circuit and the metal foil for an outer layer circuit. The metal foil for an outer layer circuit is further subjected to etching treatment for the formation of the outer layer circuit to produce the printed circuit board.

The printed circuit board obtained by the production example described above has an insulating layer and a conductor layer formed on a surface of this insulating layer and is configured such that the insulating layer comprises the resin composition of the present embodiment mentioned above. Specifically, the prepreg of the present embodiment mentioned above (the base material and the resin composition of the present embodiment with which the base material is impregnated or coated) and the layer of the resin composition (the layer consisting of the resin composition of the present embodiment) in the metal foil-clad laminate of the present embodiment mentioned above are each constituted by the insulating layer comprising the resin composition of the present embodiment.

The resin sheet of the present embodiment can be obtained by coating a support with a solution of the resin composition dissolved in a solvent, followed by drying. In this context, examples of the support used include, but are not particularly limited to: organic film base materials such as polyethylene films, polypropylene films, polycarbonate films, polyethylene terephthalate films, ethylene-tetrafluoroethylene copolymer films, and mold release films comprising these films surface-coated with a mold release agent, and polyimide films; and plate-like supports such as conductor foil (e.g., copper foil and aluminum foil), glass plates, SUS plates, and FRP. Examples of the coating method include a method of coating the support with the solution of the resin composition of the present embodiment dissolved or compatibilized in a solvent using a bar coater, a die coater, a doctor blade, a baker applicator, or the like to prepare a laminated sheet integrally composed of the support and the resin sheet. After coating and subsequent drying, the support may be removed from the resulting laminated sheet by peeling or etching to obtain a monolayer sheet (resin sheet). The monolayer sheet (resin sheet) can also be obtained without the use of the support by supplying the solution of the resin composition of the present

embodiment dissolved or compatibilized in a solvent into a mold having a sheet-like cavity, followed by drying or the like to form a sheet shape.

In the preparation of the resin sheet (monolayer or laminated sheet) of the present embodiment, the drying conditions for removing the solvent are not particularly limited and preferably involve a temperature of 20°C to 200°C for 1 to 90 minutes because a lower temperature is likely to cause the solvent to remain in the resin composition and a higher temperature facilitates the progression of cure of the resin composition. The thickness of the resin layer in the resin sheet (monolayer or laminated sheet) of the present embodiment can be adjusted by the concentration of the solution of the resin composition of the present embodiment and a coating thickness and is not particularly limited. The thickness is preferably 0.1 to 500 μm because a larger coating thickness is generally likely to cause the solvent to remain during drying.

The fiber-reinforced composite material of the present embodiment comprises the resin composition and can be produced using the resin composition and reinforcing fiber. The reinforcing fiber contained in the fiber-reinforced composite material is, for example, fiber such as carbon fiber, glass fiber, aramide fiber, boron fiber, PBO fiber, high-strength polyethylene fiber, alumina fiber, or silicon carbide fiber can be used. The form or sequence of the reinforcing fiber is not particularly limited and can be appropriately selected from, for example, woven fabrics, unwoven fabrics, mats, knits, braided cords, unidirectional strands, rovings, and chopped strands. Alternatively, a preform (laminated woven ground fabrics made of the reinforcing fiber, or an integrally sutured product thereof using stitching yarn, or a fiber structure such as a three-dimensional woven fabric or a knitted or braided fabric) may be applied to the form of the reinforcing fiber. Specific examples of methods for producing these fiber-reinforced composite materials include liquid composite molding methods, resin film infusion methods, filament winding methods, hand lay-up methods, and pultrusion methods. Among these methods, a resin transfer molding method, which is one of the liquid composite molding methods, is adaptable to various purposes because materials other than preforms, such as a metal plate, a foam core, and a honeycomb core can be loaded in molds in advance. Therefore, the resin transfer molding method is preferably used in the largescale production of composite materials having a relatively complicated shape in a short time.

The adhesive of the present embodiment comprises the resin composition and can be produced using the resin composition. A method generally known in the art can be appropriately applied to a method for producing the adhesive and is not particularly limited. For example, the resin composition can be mixed with various additives or solvents, etc. known to be used for the production of adhesives, using a mixer known in the art to produce the adhesive. For the mixing, a method generally known in the art can be appropriately applied to a method for adding the resin composition, various additives, and solvents and is not particularly limited.

The resin composition according to the present embodiment has excellent low thermal expansibility, flame retardance, and heat resistance and is therefore very useful as a highly functional polymer material. The resin composition of the present embodiment is preferably used as a material excellent thermally, electrically, and in mechanical properties in electrical insulating materials, materials for encapsulation, adhesives, laminating materials, resists, and buildup laminate materials as well as fixation materials, structural members, reinforcing agents, templating materials, etc. in fields such as civil engineering and construction, electrical and electronics, automobiles, railroads, shipping, aircrafts, sport goods, and arts and crafts. Among others, the resin composition of the present embodiments is suitable for electrical insulating materials, semiconductor encapsulation materials, adhesives for electronic components, aircraft structural members, satellite structural members, and railroad vehicle structural members, which are required to have low thermal expansibility, flame retardance, and a high level of mechanical strength.

## Examples

Hereinafter, the present embodiment will be described more specifically with reference to Examples. However, the present invention is not intended to be limited by Examples below.

(Measurement of OH group (g/eq.) equivalent of hydroxy group-containing aromatic compound)

According to JIS-K0070, the OH group equivalent (g/eq.) was determined by the pyridine-acetyl chloride method.

(Measurement of weight-average molecular weight (Mw) of cyanic acid ester compound)

10 μL of a solution containing 1 g of the cyanic acid ester compound dissolved in 100 g of tetrahydrofuran (solvent) was injected to high-performance liquid chromatography (high-performance liquid chromatograph LaChrom(R) Elite manufactured by Hitachi High-Technologies Corp.), and analysis was carried out. Column: two TSKgel $GMH_{HR}$-M (30 cm long × 7.8 mm in inside diameter) columns manufactured by Tosho Corp. Mobile phase: tetrahydrofuran. Flow rate: 1 mL/min. Detector: RI. The weight-average molecular weight Mw was determined by GPC with polystyrene as a standard.

[Example 1] Synthesis of cyanic acid ester compound of thiophene-containing phenol novolac resin (hereinafter, abbreviated to TiPRCN)

TiPRCN represented by the following formula (1) was synthesized as mentioned later.

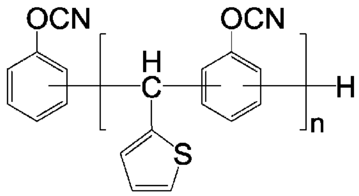 (1)

wherein n represents an integer of 2 to 10.

<Synthesis of thiophene-containing phenol novolac resin (hereinafter, abbreviated to "TiPROH")>

First, TiPROH represented by the following formula (2) was synthesized by a method described below.

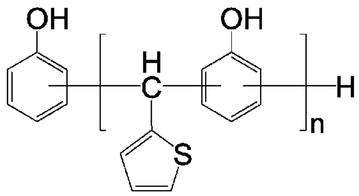 (2)

wherein n represents an integer of 2 to 10.

113 g of phenol, 28 g of methanol, and 12 g of sodium hydroxide were added, stirred, dissolved, and then heated to reflux. 33.8 g of 2-thiophene aldehyde was added dropwise over 2 hours to the reaction solution in the refluxed state. Then, the reaction solution was reacted at a reflux temperature (90 to 100°C) for 20 hours and then neutralized with 30 g of an aqueous solution containing 35% by mass of hydrochloric acid, and 5 g of an aqueous solution containing 80% by mass of hydrazine was added thereto. Subsequently, 150 g of methyl isobutyl ketone was added thereto. After repetitive washing with water, unreacted phenol and methyl isobutyl ketone were distilled off under reduced pressure by heating to obtain 373 g of TiPROH. The obtained TiPROH had an OH group equivalent of 156 g/eq.

<Synthesis of TiPRCN>

Next, 20 g (OH group equivalent: 156 g/eq.) (0.128 mol based on OH groups) of TiPROH (weight-average molecular weight (Mw): 540; the GPC chart is shown in Figure 1) obtained by the method described above and 9.8 g (0.096 mol) of triethylamine (0.75 mol with respect to 1 mol of the hydroxy groups) were dissolved in 100 g of dichloromethane to prepare solution 1.

To 12.6 g (0.205 mol) (1.60 mol with respect to 1 mol of the hydroxy groups) of cyanogen chloride, 29.5 g of dichloromethane, 20.1 g (0.199 mol) (1.55 mol with respect to 1 mol of the hydroxy groups) of 36% by mass of hydrochloric acid, and 124.8 g of water, the solution 1 was poured over 10 minutes with stirring while the temperature of the solution was kept at -6 to -3°C. After the completion of the pouring of the solution 1, the mixture was stirred at the same temperature as above for 30 minutes. Then, a solution containing 17.7 g (0.173 mol) (1.35 mol with respect to 1 mol of the hydroxy groups) of triethylamine dissolved in 17.7 g of dichloromethane (solution 2) was poured thereto over 15 minutes. After the completion of the pouring of the solution 2, the mixture was stirred at the same temperature as above for 30 minutes to complete the reaction.

Then, the reaction solution was left standing to separate an organic phase from an aqueous phase. The obtained organic phase was washed with 60 mL of 0.1 N hydrochloric acid and then washed with 60 g of water four times. The electroconductivity of a waste liquid from the fourth washing with water was 20 μS/cm to confirm that removable ionic compounds were fully removed by the washing with water.

The organic phase thus washed with water was concentrated under reduced pressure and finally concentrated to dryness at 90°C for 1 hour to obtain 22.7 g of the cyanic acid ester compound TiPRCN (brown viscous substance) of interest. The obtained cyanic acid ester compound TiPRCN had a weight-average molecular weight (Mw) of 600. The GPC chart is shown in Figure 2. The IR (infrared absorption) spectrum of TiPRCN measured by FT-IR exhibited absorption of 2235 $cm^{-1}$ and 2262 $cm^{-1}$ (cyanic acid ester group) and did not exhibit the absorption of hydroxy groups. The IR chart is

shown in Figure 3. TiPRCN was able to be dissolved at 50% by mass or more at 25°C in methyl ethyl ketone. The compound of the formula (1) was a mixture of compounds wherein n was in the range of 2 to 5.

[Example 2]

<Preparation of curable resin composition and preparation of cured product>

100 parts by mass of the cyanic acid ester compound TiPRCN obtained in Example 1 were added to an eggplantshaped flask, molten by heating at 150°C, and deaerated using a vacuum pump. Then, the solution was injected into the mold described in JIS-K7238-2-2009, placed in an oven, and cured by heating at 180°C for 3 hours and then at 250°C for 3 hours to obtain a cured product of 100 mm in one side and 2 mm in thickness.

(Comparative Example 1)

A cured product was obtained in the same way as in Example 1 except that 100 parts by mass of 2,2-bis(4-cyanatophenyl)propane (trade name: Skylex(R), manufactured by Mitsubishi Gas Chemical Co., Inc.; abbreviated to TA) were used instead of 100 parts by mass of TiPRCN in Example 1. The 2,2-bis(4-cyanatophenyl)propane (Skylex) was able to be dissolved at 50% by mass or more at 25°C in methyl ethyl ketone.

[Physical property evaluation]

The properties of each cured product obtained as described above were evaluated by the methods given below.

<Glass transition temperature (Tg, °C)>

According to JIS-K7244-3 (JIS C6481), the dynamic viscoelasticity of the cured product was measured using a dynamic viscoelasticity measurement apparatus (model "AR2000" manufactured by TA Instruments Japan Inc.) under conditions involving a start temperature of 30°C, an end temperature of 400°C, a temperature increase rate of 3°C/min, and a measurement frequency of 1 Hz. The largest value of a loss elastic modulus (E") obtained in this measurement was used as the glass transition temperature. The glass transition temperature serves as an index for heat resistance.

<Coefficient of thermal expansion(ppm/°C)>

According to JIS-K-7197-2012 (JIS C6481), a 5 mm × 5 mm × 2 mm test specimen of the cured product was thermomechanically analyzed using a thermomechanical analysis apparatus (trade name "TMA/SS7100" manufactured by SII Nanotechnology Inc.) on the swelling/compression mode under conditions involving a start temperature of 30°C, an end temperature of 330°C, a temperature increase rate of 10°C/min, and a load of 0.05 N (49 mN) to measure the average amount of thermal expansion per °C at 60 to 120°C.

<Rate of decrease in mass (%)>

According to JIS-K7120-1987, the mass of a 3 mm × 3 mm × 2 mm test specimen was measured in a nitrogen atmosphere using a thermogravimetry-differential thermal analysis apparatus (trade name "Thermo plus EVO TG8120" manufactured by Rigaku Corp.) under conditions involving a start temperature of 40°C, an end temperature of 500°C, and a temperature increase rate of 10°C/min. The rate of decrease in mass at 500°C was calculated according to the following expression:

$$\text{Rate of decrease in mass (\%)} = (D - E) / D \times 100$$

D represents the mass at the start temperature, and E represents the mass at 500°C.

In this context, the flame retardance used in the present embodiment is defined as a large amount of residues from thermal decomposition, i.e., a low rate of decrease in mass.

<Rate of decrease in mass (%) at high temperature over long period (hereinafter, long-term heating test)>

The cured product prepared by the method described above was stored at 250°C for 360 hours in an air atmosphere in a hot-air oven. The mass after the storage was measured, and the rate of decrease in mass in the long-term heating test was calculated according to the following expression:

$$\text{Rate of decrease in mass (\%)} = (F - G) / F \times 100$$

F represents the mass before the long-term heating test, and G represents the mass after the long-term heating test.

The evaluation results are shown in Table 1.

[Table 1]

| | | | Example 2 | Comparative Example 1 |
|---|---|---|---|---|
| Formulation | TiPRCN | part by mass | 100 | 0 |
| | TA | | 0 | 100 |
| Monomer physical property | MEK solubility | % by mass | >50 | >50 |
| Physical property of cured product | Tg | °C | 330 | 300 |
| | Coefficient of thermal expansion | ppm/°C | 53 | 59 |
| | Rate of decrease in mass | % | 28 | 44 |
| | Rate of decrease in mass in long-term heating test | % | 7.7 | 20 |

As is also evident from Table 1, the cyanic acid ester compound of the thiophene-containing phenol novolac resin of the present embodiment was confirmed to have excellent solvent solubility and also have excellent handleability. The cured product of a curable resin composition containing the cyanic acid ester compound of the present embodiment was confirmed to have a low rate of thermal expansion and excellent flame retardance and heat resistance as compared with a cured product obtained using the conventional cyanic acid ester compound.

[Example 3]

50 parts by mass of the cyanic acid ester compound TiPRCN obtained by Example 1, 50 parts by mass of a biphenyl aralkyl-based epoxy resin (trade name "NC-3000-FH", manufactured by Nippon Kayaku Co., Ltd.), and 100 parts by mass of molten silica (trade name "SC2050MB", manufactured by Admatechs Co., Ltd.) were mixed to obtain varnish. This varnish was diluted with methyl ethyl ketone. Then, a woven fabric of E glass having a thickness of 0.1 mm was impregnated and coated with the dilution and further dried by heating at 165°C for 4 minutes to obtain a prepreg having a resin content of 50% by mass.

12 μm thick electrolytic copper foil (trade name "3EC-M3-VLP", manufactured by Mitsui Mining & Smelting Co., Ltd.) was disposed both above and below eight layers of the obtained prepreg in the direction of lamination, followed by lamination molding at a pressure of 40 $kgf/cm^2$ and a temperature of 230°C for 120 minutes to obtain a copper-clad laminate (metal foil-clad laminate) having an insulating layer thickness of 0.8 mm. The obtained copper-clad laminate was used in the evaluation of peel strength, a glass transition temperature, the rate of thermal expansion, the rate of water absorption, and thermal conductivity as described below. The results are shown in Table 2.

[Comparative Example 2]

A copper-clad laminate having a thickness of 0.8 mm was obtained in the same way as in Example 3 except that 50 parts by mass of a bisphenol A-based cyanic acid ester compound (trade name "CA210", manufactured by Mitsubishi Gas Chemical Co., Inc.) were used instead of 50 parts by mass of TiPRCN in Example 3. Results of evaluating the obtained copper-clad laminate are shown in Table 2.

[Measurement method and evaluation method]

<Copper foil peel strength (kN/m)>

A test specimen with 12 μm metal foil (30 mm × 150 mm × 0.8 mm) was prepared from the obtained metal foil-clad laminate having an insulating layer thickness of 0.8 mm. According to JIS C6481, the peel strength of the copper foil

was measured (N = 3), and an average value of the lowest values was used as a measurement value.

<Glass transition temperature (°C)>

The copper foil on the surface of the obtained metal foil-clad laminate having an insulating layer thickness of 0.8 mm was removed by etching to obtain a sample for measurement. According to JIS C6481, the glass transition temperature of this sample for measurement was measured by the DMA method using a dynamic viscoelasticity analysis apparatus (manufactured by TA Instruments Japan Inc.).

<Coefficient of thermal expansion (ppm/°C)>

The copper foil on the surface of the obtained metal foil-clad laminate having an insulating layer thickness of 0.8 mm was removed by etching to obtain a sample for measurement. The coefficient of thermal expansion of the insulating layer of the laminate in the thickness direction of the glass cloth was measured by the TMA (thermo-mechanical analysis) method defined by JIS C6481 using the sample for measurement to determine the value. Specifically, the copper foil on both sides of the metal foil-clad laminate obtained as described above was removed by etching. Then, the linear coefficient of thermal expansion (ppm/°C) was measured at 60°C to 120°C in a thermomechanical analysis apparatus (manufactured by TA Instruments Japan Inc.) at a temperature increase rate of 10°C/min from 40°C to 340°C.

<Rate of water absorption (%)>

A 50 mm × 50 mm sample was prepared using the obtained metal foil-clad laminate having an insulating layer thickness of 0.8 mm, and the copper foil on the surface of the sample was removed by etching to obtain a sample for measurement. According to JIS C6481, this sample for measurement was treated at 121°C and 2 atm for 1, 3, or 5 hours using a pressure cooker tester (model PC-3, manufactured by Hirayama Manufacturing Corp.). Then, the rate of water absorption was measured.

<Thermal conductivity (W/m·K) >

The copper foil on the surface of the obtained metal foil-clad laminate having an insulating layer thickness of 0.8 mm was removed by etching to obtain a sample for measurement. The density of this sample for measurement was measured, and the specific heat was also measured by DSC (model Q100, manufactured by TA Instruments Japan Inc.). The rate of thermal diffusion was further measured using a xenon flash analyzer (Bruker: LFA 447 Nanoflash). The thermal conductivity was calculated according to the following expression:

Thermal conductivity (W/m·K) = Density (kg/$m^3$) × Specific heat (kJ/kg·K) × Rate of thermal diffusion ($m^2$/S) × 1000

[Table 2]

| | | Example 3 | Comparative Example 2 |
|---|---|---|---|
| Peel strength | kN/m | 0.96 | 0.78 |
| Glass transition temperature | °C | 296 | 273 |
| Coefficient of thermal expansion (z direction) | ppm/°C | 21.4 | 23.3 |
| Rate of water absorption | % | 0.30 | 0.42 |
| Thermal conductivity | W/m·K | 0.73 | 0.67 |

As is evident from Table 2, use of the resin composition according to the present invention was confirmed to be able to realize a prepreg and a printed circuit board, etc. also excellent in peel strength, glass transition temperature, rate of thermal expansion, rate of water absorption, and thermal conductivity.

[Example 4]

50 parts by mass of the cyanic acid ester compound TiPRCN obtained by Example 1, 50 parts by mass of a maleimide compound represented by the formula (8) (BMI-2300, maleimide equivalent: 275 g/eq., manufactured by

Daiwa Fine Chemicals Co., Ltd.), and 100 parts by mass of molten silica (SC2050MB, manufactured by Admatechs Co., Ltd.) were mixed to obtain varnish. This varnish was diluted with methyl ethyl ketone. A woven fabric of E glass having a thickness of 0.1 mm was impregnated and coated with the dilution and dried by heating at 165°C for 3 minutes to obtain a prepreg having a resin content of 50% by mass.

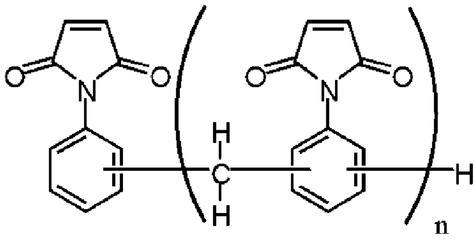 (8)

wherein n represents an integer of 2 to 3.

12 μm thick electrolytic copper foil (3EC-M3-VLP, manufactured by Mitsui Mining & Smelting Co., Ltd.) was disposed above and below eight layers of the obtained prepreg, followed by lamination molding at a pressure of 40 $kgf/cm^2$ and a temperature of 230°C for 120 minutes to obtain a metal foil-clad laminate having an insulating layer thickness of 0.8 mm. The obtained metal foil-clad laminate was used in the evaluation of a glass transition temperature, the coefficient of thermal expansion, copper foil peel strength and thermal conductivity. The results are shown in Table 3.

[Comparative Example 3]

A metal foil-clad laminate having a thickness of 0.8 mm was obtained in the same way as in Example 4 except that 50 parts by mass of a bisphenol A-based cyanic acid ester compound (trade name "CA210", manufactured by Mitsubishi Gas Chemical Co., Inc.) were used instead of 50 parts by mass of TiPRCN obtained by Example 1, in Example 4. Results of evaluating the obtained metal foil-clad laminate are shown in Table 3.

[Measurement method and evaluation method]

<Glass transition temperature (°C)>

The glass transition temperature was measured by the method mentioned above.

<Coefficient of thermal expansion (ppm/°C)>

The coefficient of thermal expansion was measured by the method mentioned above.

<Flexural modulus (GPa)>

The copper foil on both sides of the obtained metal foil-clad laminate having an insulating layer thickness of 0.8 mm was removed by etching. Then, according to JIS C6481, the flexural modulus of a test specimen (50 mm × 25 mm × 0.8 mm) was measured (N = 5), and an average value of the largest values was used as a measurement value.

<Thermal conductivity (W/m·K)>

The thermal conductivity was measured by the method mentioned above.

[Table 3]

| | | Example 4 | Comparative Example 3 |
|---|---|---|---|
| Glass transition temperature | [°C] | 314 | 283 |
| Coefficient of thermal expansion | [ppm/°C] | 15.3 | 18.9 |
| Flexural modulus | [GPa] | 36.1 | 33.0 |
| Thermal conductivity | [W/m·K] | 0.74 | 0.64 |

As is evident from Table 3, use of the resin composition according to the present invention was confirmed to be

able to realize a prepreg and a printed circuit board, etc. having a high glass transition temperature and low thermal expansibility and also having excellent flexural modulus and thermal conductivity.

Industrial Applicability

As described above, the resin composition of the present embodiment can be utilized widely and effectively for various purposes such as electric and electronic materials, machine tool materials, and aeronautical materials, for example, as an electrical insulating material, a semiconductor plastic package, an encapsulation material, an adhesive, a laminating material, a resist, or a buildup laminate material. Particularly, the resin composition of the present embodiment can be very effectively utilized as a material for recent printed circuit boards adaptable to high integration and high density, such as information terminal equipment and communication equipment.

The laminate and the metal foil-clad laminate, etc. of the present embodiment not only has heat resistance and flame retardance but has excellent performance in terms of moisture-absorbing heat resistance. Therefore, its industrially practical use is very high.

The laminate and the metal foil-clad laminate, etc. of the present embodiment not only has a high glass transition temperature and low thermal expansibility but has excellent performance in terms of metal adhesion and thermal conductivity. Therefore, its industrially practical use is very high.

## Claims

1. A cyanic acid ester compound having a structure represented by the following general formula (1):

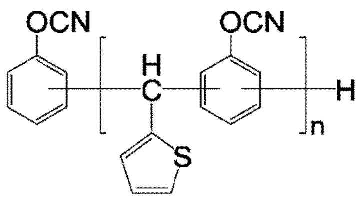

(1)

wherein n represents an integer of 2 to 10.

2. The cyanic acid ester compound according to claim 1, wherein the cyanic acid ester compound has a weight-average molecular weight Mw of 100 to 3500.

3. The cyanic acid ester compound according to claim 1 or 2, wherein the cyanic acid ester compound is a cyanation product of a thiophene-containing phenol novolac resin represented by the following formula (2):

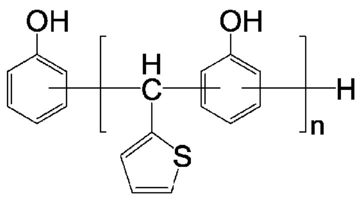

(2)

wherein n represents an integer of 2 to 10.

4. A method for producing a cyanic acid ester compound, comprising a cyanation step of cyanating a thiophene-containing phenol novolac resin to obtain a cyanic acid ester compound having a structure represented by the following general formula (1):

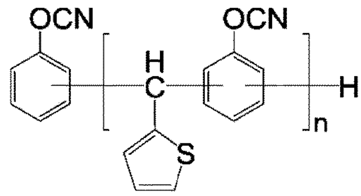 (1)

wherein n represents an integer of 2 to 10.

5. The method for producing a cyanic acid ester compound according to claim 4, wherein the method further comprises, before the cyanation step, the step of reacting 2-thiophene aldehyde with phenol in the presence of a basic catalyst to obtain the thiophene-containing phenol novolac resin.

6. A resin composition comprising a cyanic acid ester compound according to any one of claims 1 to 3.

7. The resin composition according to claim 6, wherein a content of the cyanic acid ester compound is 1 to 90 parts by mass with respect to 100 parts by mass of a resin solid in the resin composition.

8. The resin composition according to claim 6 or 7, wherein the resin composition further comprises one or more components selected from the group consisting of a cyanic acid ester compound other than the cyanic acid ester compound, a maleimide compound, a phenol resin, an epoxy resin, an oxetane resin, a benzoxazine compound, and a compound having a polymerizable unsaturated group.

9. The resin composition according to claim 8, wherein the resin composition comprises an epoxy resin.

10. The resin composition according to claim 8 or 9, wherein the resin composition comprises a maleimide compound.

11. The resin composition according to any one of claims 6 to 10, wherein the resin composition further comprises a filler.

12. The resin composition according to claim 11, wherein a content of the filler is 50 to 1600 parts by mass with respect to 100 parts by mass of a resin solid in the resin composition.

13. A cured product obtained by curing a resin composition according to any one of claims 6 to 12.

14. A prepreg for a structural material obtained by impregnating or coating a base material with a resin composition according to any one of claims 6 to 12, followed by drying.

15. A material for encapsulation comprising a resin composition according to any one of claims 6 to 12.

16. A fiber-reinforced composite material comprising a resin composition according to any one of claims 6 to 12.

17. An adhesive comprising a resin composition according to any one of claims 6 to 12.

18. A prepreg comprising a base material, and a resin composition according to any one of claims 6 to 12 with which the base material is impregnated or coated.

19. A metal foil-clad laminate comprising at least one or more layers of a prepreg according to claim 18, and metal foil disposed on one side or both sides of the prepreg.

20. A resin sheet comprising a resin composition according to any one of claims 6 to 12.

21. A printed circuit board comprising an insulating layer, and a conductor layer formed on a surface of the insulating layer, wherein the insulating layer comprises a resin composition according to any one of claims 6 to 12.

**Patentansprüche**

1. Cyansäureesterverbindung mit einer Struktur, dargestellt durch die folgende allgemeine Formel (1):

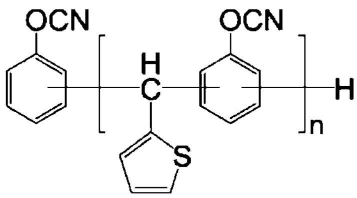

(1)

wobei n eine ganze Zahl von 2 bis 10 darstellt.

2. Cyansäureesterverbindung gemäß Anspruch 1, wobei die Cyansäureesterverbindung ein gewichtsmittleres Molekulargewicht Mw von 100 bis 3500 aufweist.

3. Cyansäureesterverbindung gemäß Anspruch 1 oder 2, wobei die Cyansäureesterverbindung ein Cyanierungsprodukt eines thiophenhaltigen Phenol-Novolak-Harzes ist, dargestellt durch die folgende Formel (2):

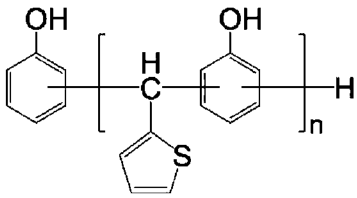

(2)

wobei n eine ganze Zahl von 2 bis 10 darstellt.

4. Verfahren zur Herstellung einer Cyansäureesterverbindung, umfassend einen Cyanierungsschritt, bei dem ein thiophenhaltiges Phenol-Novolak-Harz cyaniert wird, um eine Cyansäureesterverbindung mit einer Struktur zu erhalten, die durch die folgende allgemeine Formel (1) dargestellt wird:

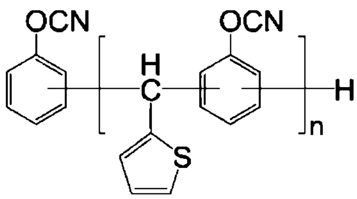

(1)

wobei n eine ganze Zahl von 2 bis 10 darstellt.

5. Verfahren zur Herstellung einer Cyansäureesterverbindung gemäß Anspruch 4, wobei das Verfahren vor dem Cyanierungsschritt ferner umfasst den Schritt, 2-Thiophenaldehyd mit Phenol in Gegenwart eines basischen Katalysators zu reagieren, um das thiophenhaltige Phenol-Novolak-Harz zu erhalten.

6. Harzzusammensetzung, die eine Cyansäureesterverbindung gemäß mindestens einem der Ansprüche 1 bis 3 umfasst.

7. Harzzusammensetzung gemäß Anspruch 6, wobei der Gehalt an Cyansäureesterverbindung 1 bis 90 Masseteile, bezogen auf 100 Masseteile Harzfeststoff in der Harzzusammensetzung, beträgt.

8. Harzzusammensetzung gemäß Anspruch 6 oder 7, wobei die Harzzusammensetzung ferner umfasst eine oder mehrere Komponenten, ausgewählt aus der Gruppe, bestehend aus einer anderen Cyansäureesterverbindung als der Cyansäureesterverbindung, einer Maleimidverbindung, einem Phenolharz, einem Epoxidharz, einem Oxetanharz, einer Benzoxazinverbindung und einer Verbindung mit einer polymerisierbaren ungesättigten Gruppe.

9. Harzzusammensetzung gemäß Anspruch 8, wobei die Harzzusammensetzung ein Epoxidharz umfasst.

10. Harzzusammensetzung gemäß Anspruch 8 oder 9, wobei die Harzzusammensetzung eine Maleimidverbindung umfasst.

11. Harzzusammensetzung gemäß mindestens einem der Ansprüche 6 bis 10, wobei die Harzzusammensetzung ferner einen Füllstoff umfasst.

12. Harzzusammensetzung gemäß Anspruch 11, wobei der Gehalt des Füllstoffs 50 bis 1600 Masseteile, bezogen auf 100 Masseteile eines Harzfeststoffs in der Harzzusammensetzung, beträgt.

13. Ausgehärtetes Produkt, das durch Aushärten einer Harzzusammensetzung gemäß mindestens einem der Ansprüche 6 bis 12 erhalten wird.

14. Prepreg für ein Strukturmaterial, das erhalten wird durch Imprägnieren oder Beschichten eines Basismaterials mit einer Harzzusammensetzung gemäß mindestens einem der Ansprüche 6 bis 12, und anschließendes Trocknen.

15. Material zum Einkapseln, umfassend eine Harzzusammensetzung gemäß mindestens einem der Ansprüche 6 bis 12.

16. Faserverstärkter Verbundwerkstoff, umfassend eine Harzzusammensetzung gemäß mindestens einem der Ansprüche 6 bis 12.

17. Klebstoff, umfassend eine Harzzusammensetzung gemäß mindestens einem der Ansprüche 6 bis 12.

18. Prepreg, umfassend ein Basismaterial, und eine Harzzusammensetzung gemäß mindestens einem der Ansprüche 6 bis 12, mit der das Basismaterial imprägniert oder beschichtet ist.

19. Metallfolienplattiertes Laminat (metal foil-clad laminate) umfassend mindestens eine oder mehrere Schichten eines Prepregs gemäß Anspruch 18, und Metallfolie, die auf einer Seite oder beiden Seiten des Prepregs angeordnet ist.

20. Harzfolie, umfassend eine Harzzusammensetzung gemäß mindestens einem der Ansprüche 6 bis 12.

21. Leiterplatte, umfassend eine Isolierschicht, und eine auf einer Oberfläche der Isolierschicht ausgebildete Leiterschicht, wobei die Isolierschicht umfasst eine Harzzusammensetzung gemäß mindestens einem der Ansprüche 6 bis 12.

**Revendications**

1. Composé d'ester d'acide cyanique présentant une structure représentée par la formule générale suivante (1) :

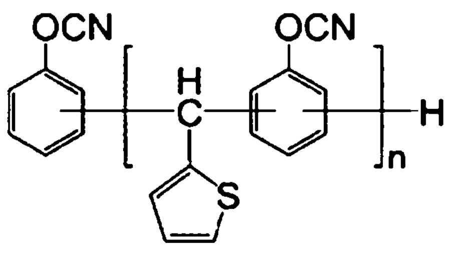 (1)

dans laquelle n représente un nombre entier de 2 à 10.

2. Composé d'ester d'acide cyanique selon la revendication 1, dans lequel le composé d'ester d'acide cyanique présente un poids moléculaire moyen en poids Mw allant de 100 à 3500.

3. Composé d'ester d'acide cyanique selon la revendication 1 ou la revendication 2, dans lequel le composé d'ester d'acide cyanique étant un produit de cyanation d'une résine novolaque de phénol contient du thiophène représentée

par la formule (2) suivante :

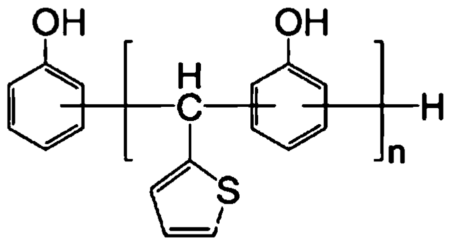 (2)

dans laquelle n représente un nombre entier de 2 à 10.

4. Procédé de production d'un composé d'ester d'acide cyanique, comprenant une étape de cyanation consistant à cyanater une résine novolaque de phénol contenant du thiophène pour obtenir un composé d'ester d'acide cyanique présentant une structure représentée par la formule générale suivante (1) :

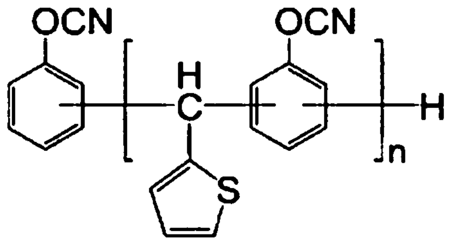 (1)

dans laquelle n représente un nombre entier de 2 à 10.

5. Procédé de production d'un composé d'ester d'acide cyanique selon la revendication 4, dans lequel le procédé comprend en outre, avant l'étape de cyanation, l'étape consistant à faire réagir le 2-thiophene aldehyde avec du phénol en présence d'un catalyseur basique pour obtenir la résine novolaque de phénol contenant du thiophène.

6. Composition de résine comprenant un composé d'ester d'acide cyanique selon l'une quelconque des revendications 1 à 3.

7. Composition de résine selon la revendication 6, dans laquelle une teneur du composé d'ester d'acide cyanique est de 1 à 90 parties en masse par rapport à 100 parties en masse d'un solide de résine dans la composition de résine.

8. Composition de résine selon la revendication 6 ou la revendication 7, dans laquelle la composition de résine comprend en outre un ou plusieurs composants sélectionnés dans le groupe consistant en un composé d'ester d'acide cyanique autre que le composé d'ester d'acide cyanique, un composé maléimide, une résine phénolique, une résine époxy, une résine oxétane, un composé benzoxazine, et un composé présentant un groupe insaturé polymérisable.

9. Composition de résine selon la revendication 8, dans laquelle la composition de résine comprend une résine époxy.

10. Composition de résine selon la revendication 8 ou la revendication 9, dans laquelle la composition de résine comprend un composé maléimide.

11. Composition de résine selon l'une quelconque des revendications 6 à 10, dans laquelle la composition de résine comprend en outre une charge.

12. Composition de résine selon la revendication 11, dans laquelle une teneur en charge est de 50 à 1600 parties en masse par rapport à 100 parties en masse d'un solide de résine dans la composition de résine.

13. Produit durci obtenu par durcissement d'une composition de résine selon l'une quelconque des revendications 6 à 12.

14. Préimprégné pour un matériau structurel obtenu en imprégnant ou en enduisant un matériau de base avec une

composition de résine selon l'une quelconque des revendications 6 à 12, suivi d'un séchage.

**15.** Matériau d'encapsulation comprenant une composition de résine selon l'une quelconque des revendications 6 à 12.

**16.** Matériau composite renforcé par des fibres, comprenant une composition de résine selon l'une quelconque des revendications 6 à 12.

**17.** Adhésif comprenant une composition de résine selon l'une quelconque des revendications 6 à 12.

**18.** Préimprégné comprenant un matériau de base et une composition de résine selon l'une quelconque des revendications 6 à 12, avec laquelle le matériau de base est imprégné ou enduit.

**19.** Stratifié revêtu d'une feuille métallique comprenant au moins une ou plusieurs couches d'un préimprégné selon la revendication 18, et une feuille métallique disposée sur un côté ou à la fois sur les deux côtés du préimprégné.

**20.** Feuille de résine comprenant une composition de résine selon l'une quelconque des revendications 6 à 12.

**21.** Carte de circuit imprimé comprenant une couche isolante, et une couche conductrice formée sur une surface de la couche isolante, dans laquelle la couche isolante comprend une composition de résine selon l'une quelconque des revendications 6 à 12.

Figure 1

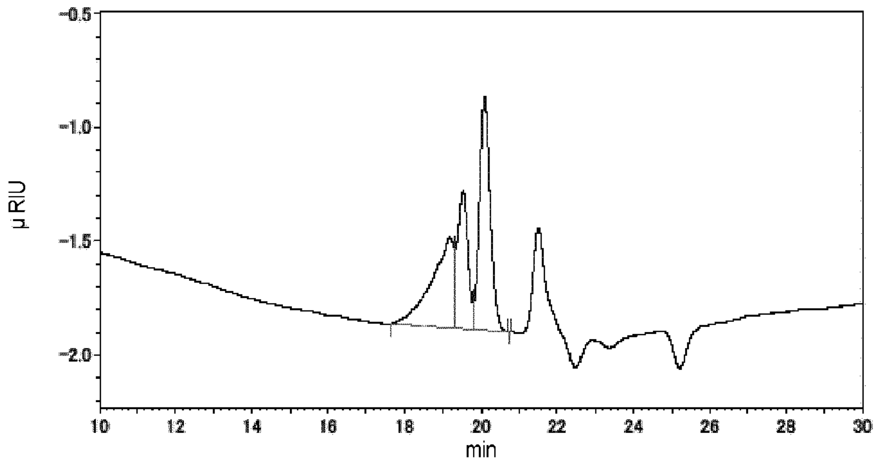


| Retention time | Area% |
|---|---|
| 19.2min. | 33.4% |
| 19.5min. | 24.0% |
| 20.1min. | 42.6% |

Figure 2

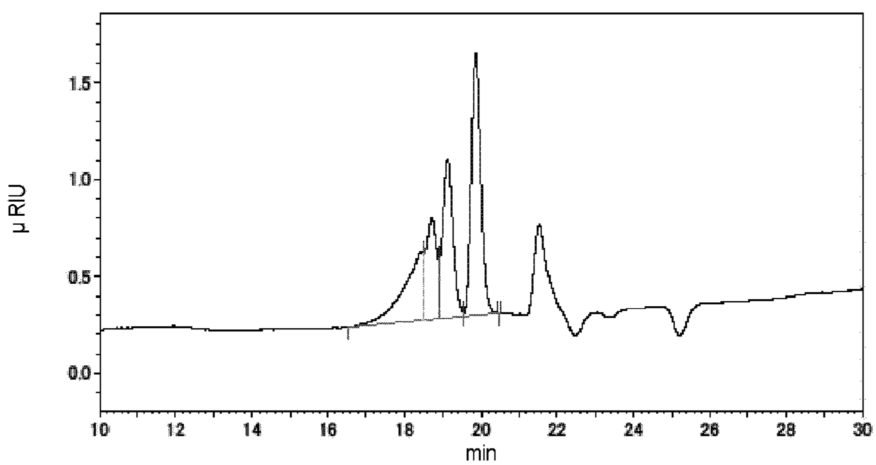


| Retention time | Area% |
|---|---|
| 18.4min. | 20.4% |
| 18.7min. | 16.3% |
| 19.1min. | 25.3% |
| 19.9min. | 38.0% |

Figure 3

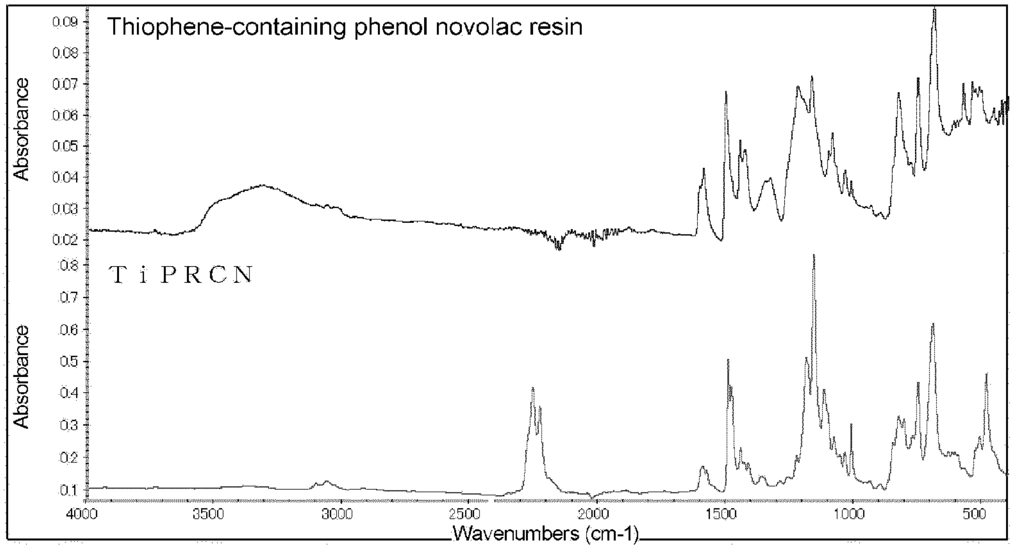
Thiophene-containing phenol novolac resin
Absorbance
0.09
0.08
0.07
0.06
0.05
0.04
0.03
0.02
T i P R C N
Absorbance
0.8
0.7
0.6
0.5
0.4
0.3
0.2
0.1
4000
3500
3000
2500
2000
1500
1000
500
Wavenumbers (cm-1)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2671904 A1
- EP 1942129 A1
- WO 2012057144 A
- JP 4407823 B
- JP 4654770 B
- JP 2012036114 A
- JP 5104312 B
- WO 201321869 A
- JP 2613056 B
- JP 2010180147 A
- JP 2013053218 A
- WO 2013065694 A
- JP 11124433 A
- WO 2014203866 A
- JP 10237060 A
- JP 4082481 B
- JP 4111410 B
- US 3553244 A
- JP 3319061 B
- JP 3905559 B
- JP 4055210 B
- JP 2991054 B
- JP 5026727 B